(19) Europäisches Patentamt

European Patent Office

Office européen des brevets

(11) **EP 3 166 578 B1**

(12) **FASCICULE DE BREVET EUROPEEN**

(45) Date de publication et mention
de la délivrance du brevet:
**18.03.2020   Bulletin 2020/12**

(21) Numéro de dépôt: **15732727.1**

(22) Date de dépôt: **01.07.2015**

(51) Int Cl.:
*A61K 8/34* *(2006.01)*          *A61K 8/60* *(2006.01)*
*A61Q 13/00* *(2006.01)*        *A61K 8/73* *(2006.01)*
*A61Q 15/00* *(2006.01)*        *A61K 8/02* *(2006.01)*
*A61K 8/04* *(2006.01)*          *A61K 8/11* *(2006.01)*

(86) Numéro de dépôt international:
**PCT/EP2015/065003**

(87) Numéro de publication internationale:
**WO 2016/005245 (14.01.2016 Gazette 2016/02)**

(54) **COMPOSITION ANHYDRE AEROSOL A BASE DE PARTICULES ENCAPSULANT UN AGENT BENEFIQUE**

WASSERFREIE AEROSOL-ZUBEREITUNG BEZOGEN AUF PARTIKELN ENTHALTEND VERKAPSELTEN PFLEGESTOFF

ANHYDROUS AEROSOL COMPOSITION BASED ON PARTICULES CONTAINING AN ENCAPSULATED BENEFIC AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **09.07.2014   FR 1456629**

(43) Date de publication de la demande:
**17.05.2017   Bulletin 2017/20**

(73) Titulaire: **L'Oréal**
**75008 Paris (FR)**

(72) Inventeurs:
• **MALLE, Gérard**
  **F-77580 Villiers S/Morin (FR)**
• **LUUKAS, Tiina**
  **F-93270 Sevran (FR)**
• **BARA, Isabelle**
  **F-94210 La Varenne St Hilaire (FR)**

(74) Mandataire: **L'Oreal**
**Service D.I.P.I.**
**9, rue Pierre Dreyfus**
**92110 Clichy (FR)**

(56) Documents cités:
**DE-A1-102008 035 014      JP-A- 2008 156 236**
**US-A- 3 971 852              US-A- 5 508 259**
**US-A1- 2004 029 750**

• **MARTIN ET AL.: "Encapsulation and Co-Precipitation Processes with Supercritical Fluids:Applications with Essential Oils", THE OPEN CHEMICAL ENGINEERING JOURNAL, vol. 4, 2010, pages 31-41, XP002737531,**

Remarques:
Le dossier contient des informations techniques présentées postérieurement au dépôt de la demande et ne figurant pas dans le présent fascicule.

**Description**

La présente invention concerne une composition anhydre comprenant :

1) au moins des particules comprenant un cœur contenant au moins un agent bénéfique et une enveloppe entourant le cœur ; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de Dextrose Equivalent allant de 4 à 20;
lesdites particules présentant simultanément une densité de poudre versée allant de 300,0g/l à 600,0g/l et une densité absolue supérieure à 1,0;
les particules étant sphériques, et ayant un diamètre moyen en nombre allant de 1 à 30µm, et un diamètre moyen en volume allant de 5 à 150µm, et
2) au moins un agent propulseur.

L'invention concerne également un procédé cosmétique de soin et/ou d'hygiène et/ou de de conditionnement et/ou de parfumage et/ou de maquillage d'une matière kératinique consistant à appliquer sur ladite matière kératinique une composition telle que définie précédemment.

L'invention concerne en outre un procédé de traitement cosmétique des odeurs corporelles et éventuellement de la transpiration humaine consistant à appliquer sur une matière kératinique une composition telle que définie précédemment comprenant au moins un actif déodorant et/ou un actif anti-transpirant sous forme libre (non encapsulé) et/ou sous forme encapsulée.

De nombreuses formes galéniques permettent de délivrer des agents bénéfiques notamment des produits cosmétiques ou pharmaceutiques, des produits dans la parfumerie, des produits vétérinaires; des produits d'hygiène et/ou le soin des animaux; des produits d'entretien ménager comme les produits pour le soin et/ou le nettoyage du linge (détachants, adoucissants, produits pour le repassage), les produits de nettoyage et/ou d'entretien des appareils électroménagers, des produits de nettoyage et/ou d'entretien des sols, carrelages, bois etc ; des produits sanitaires comme des désodorisants, des produits détartrants, des produits déboucheurs de canalisations; des produits d'entretien des matières textiles, des produits d'entretien pour la maroquinerie comme les chaussures, les semelles; des produits issus de l'industrie agro-alimentaire; des produits issus de l'agriculture; des produits phytosanitaires; des peintures; des encres; des produits d'entretien dans l'industrie automobile.

Parmi celles-ci, les aérosols sont largement utilisés. On citera notamment, les produits d'entretien ménager, les produits sanitaires, les désodorisants, les parfums d'ambiance et les produits cosmétiques comme les déodorants, les shampooings secs, les produits coiffants ou encore les huiles de massage ou de soin des cheveux.

On sait qu'il existe une nécessité dans de nombreux domaines industriels, de protéger un certain nombre de molécules fragiles ou volatiles, de contrôler leur relargage vers un milieu extérieur et de pouvoir les formuler dans des aérosols.

Un des moyens permettant d'atteindre un tel but est leur encapsulation. Cette encapsulation a pour objectif de diminuer l'évaporation et le transfert vers l'environnement de la matière active, soit au cours du stockage, soit au cours de l'élaboration des produits ou encore au cours de leur utilisation. Elle peut également permettre de rendre le matériau plus facile d'utilisation en le diluant et en favorisant sa répartition homogène au sein du support.

Notamment, la micro-encapsulation regroupe l'ensemble des technologies permettant l'enrobage ou le piégeage de principes actifs sous forme solide, liquide, ou gazeuse au sein de particules individualisées dont la taille s'échelonne entre quelques microns et quelques millimètres. Si ces microparticules sont creuses (vésiculaires), on parle de microcapsules, si elles sont pleines (matricielles) on parle de microsphères. Leur taille varie de 1 µm à plus de 1000µm. Ces microparticules peuvent être biodégradables ou non et peuvent contenir entre 5 et 90% (en masse) de substance active.

Les substances actives encapsulées sont d'origines très variées : principes actifs pharmaceutiques, cosmétiques, additifs alimentaires, produits phytosanitaires, essences parfumées, micro-organismes, cellules, ou encore catalyseurs de réaction chimique.

Tout l'intérêt des microparticules d'encapsulation réside dans la présence d'une membrane polymérique, qui isole et protège le contenu du milieu extérieur. Selon les cas, la membrane sera détruite lors de l'utilisation pour libérer son contenu (exemple : encarts publicitaires "scratch and sniff" libérant le parfum lorsqu'on écrase les microcapsules), ou bien la membrane restera présente tout le long de la libération du contenu, dont elle contrôlera la vitesse de diffusion (exemple : encapsulation de médicaments pour libération ralentie).

Les matériaux constituant la particule sont généralement des polymères d'origine naturelle ou synthétique, hydrophobes ou hydrophiles ou bien des lipides.

Les principaux procédés pour réaliser l'encapsulation de substances dans des microparticules sont la polymérisation interfaciale, la réticulation interfaciale, l'émulsion suivie d'une évaporation ou d'une extraction du solvant, la double émulsion évaporation/extraction de solvant, le spray-drying, le prilling ou la coacervation (JP 2008 156236 A; US 2004/029750 A1).

Le but de la présente invention est de trouver de nouvelles compositions anhydres aérosols comprenant un système d'encapsulation permettant :

- d'encapsuler des ingrédients particulièrement fragiles et volatiles en conservant l'exacte composition mise au point par le formulateur;
- de disposer de particules étanches contenant la totalité des ingrédients encapsulés en l'absence de stimuli, tout particulièrement pour les ingrédients odorants tels que les ingrédients de parfumerie, permettant ainsi au formulateur de combiner lesdites capsules avec un parfum libre de son choix
- de disposer de particules stables dans les milieux anhydres pouvant libérer tout ou partie de leur contenu quasi-instantanément au contact de l'eau et tout particulièrement de l'humidité atmosphérique ou de la transpiration
- de disposer de particules ayant une densité de poudre versée (encore appelée densité apparente non tassée) faible, ayant tendance à peu sédimenter, tout en ayant une densité absolue supérieure à 1,0, pour pouvoir les incorporer facilement dans des véhicules anhydres pour des aérosols;
- lesdites particules devant également être compatibles avec les gaz propulseurs et résistantes à la compression pour pouvoir être formulées en aérosol sans être endommagées.

Dans le brevet US 5 508 259, on a proposé des compositions parfumantes non aqueuses, notamment sous forme d'aérosols, comprenant des parfums encapsulés dans des particules sphériques (encore appelées capsules) solubles dans l'eau. Lesdites particules sont obtenues par des techniques d'encapsulation conventionnelles et en particulier le spray-drying d'une émulsion constituée d'un substrat solide filmogène en combinaison avec un agent émulsionnant et un mélange d'ingrédients de parfumerie. Le substrat solide filmogène est notamment choisi parmi l'acétate de polyvinyle, l'alcool polyvinylique, des dextrines, de l'amidon naturel ou modifié, les gommes végétales, les pectines, les xanthanes, les alginates, les carraghénanes ou encore les dérivés de cellulose tels que par exemple la carboxyméthylcellulose, la méthylcellulose ou l'hydroxyéthylcellulose. L'émulsion est ensuite déshydratée par un procédé classique d'atomisation (spray-drying), qui consiste, tel que décrit à l'exemple 1 dudit brevet, à la pulvériser en fines gouttelettes dans un atomiseur avec un débit de 50kg/h et une pression de 0,45 bars, au contact d'un courant d'air de 320m$^3$/h chauffé à 350°C afin d'évaporer l'eau, ce qui permet d'obtenir une poudre fine avec un diamètre de particules compris entre 20 et 80 microns et contenant 20% en poids de parfum.

Cependant, on a remarqué que les particules obtenues par ce procédé étaient fortement odorantes à l'état sec à cause de la présence de parfum libre (non encapsulé), qu'elles étaient principalement constituées d'agglomérats pouvant sédimenter dans un dispositif aérosol et gêner la bonne délivrance du produit et ne possédaient pas les caractéristiques de densité adaptées à l'objectif de l'invention.

Dans le brevet US 6 200 949, on a décrit également un procédé de formation d'une matière particulaire contenant un parfum hydrophile comprenant les étapes successives consistant à former une émulsion aqueuse de parfum contenant 40 à 60% en poids d'eau, 3 à 30% en poids de maltodextrine, 10 à 40% en poids d'amidon modifié hydrophobe, puis à la sécher par pulvérisation dans un atomiseur (courant d'air de 420m$^3$/h chauffé à 204°C) de sorte que les particules sont formées avec une taille moyenne d'environ 3 à environ 10 microns et une teneur en parfum de 15 à 50% en poids. Cependant, les particules obtenues par ce procédé sont fortement odorantes à l'état sec à cause de la présence de parfum libre (non encapsulé) et sont principalement constituées d'agglomérats pouvant sédimenter dans un dispositif aérosol et gêner la bonne délivrance du produit et ne possèdent pas les caractéristiques de densité adaptées à l'objectif de l'invention.

Il est donc très important de pouvoir disposer de particules d'encapsulation étanches qui ne libèrent leur contenu qu'à la demande (en réponse à l'humidité ambiante, notamment dans les zones climatiques humides, par exemple en réponse à la transpiration corporelle, au shampooing ou sous la douche, etc...), d'une part pour garantir la protection dans le temps de l'actif encapsulé surtout s'il est fragile et/ou volatil et d'autre part pour éviter les interactions avec les autres ingrédients de la formule. Lorsque l'agent bénéfique encapsulé est un ingrédient de parfumerie et/ou un parfum complet, il est d'autant plus important que l'encapsulation soit totale, ce qui conduit à des particules inodores en formules anhydres permettant au formulateur de les associer ou non avec n'importe quel parfum libre de son choix (identique ou différent) sans risque d'interactions ou de perturbation de la note parfumée choisie.

Dans le brevet EP1917098B1, on a proposé un procédé de préparation de particules d'encapsulation par précipitation, lequel procédé emploie :

- une émulsion pouvant être pompée comprenant (i) une phase continue contenant un solvant et un soluté formant une matrice dissout dans ledit solvant et (ii) une phase dispersée;
- un extracteur comprenant un gaz supercritique, sous-critique ou liquéfié;

ledit solvant étant sensiblement plus soluble dans l'extracteur que ledit soluté formant une matrice, et ledit procédé comprenant les étapes successives consistant à :

a) combiner l'émulsion pouvant être pompée avec l'extracteur dans des conditions de mélange;
b) permettre la formation de produits d'encapsulation particulaires dans lesquels la phase dispersée est imbriquée dans une matrice solide du soluté formant une matrice;
c) collecter les produits d'encapsulation et les séparer de l'extracteur. Il est indiqué que ce procédé peut être utilisé dans les industries pharmaceutique et agroalimentaire ainsi-que dans les domaines de l'agriculture, du «coating», des adhésifs et des catalyseurs. Il peut en particulier être utilisé pour encapsuler des actifs pharmaceutiques, des arômes, des enzymes, des colorants, des pesticides et des herbicides.

Après d'importantes recherches, la demanderesse a découvert de façon surprenante et inattendue qu'il était possible d'atteindre les objectifs tels qu'énoncés précédemment grâce à une composition anhydre aérosol contenant au moins des particules comprenant un cœur contenant au moins un agent bénéfique et une enveloppe entourant le cœur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de D.E. allant de 4 à 20; lesdites particules présentant simultanément une densité de poudre versée allant de 300,0 g/l à 600,0 g/l et une densité absolue supérieure à 1,0 ; et les particules étant sphériques, et avant un diamètre moyen en nombre allant de 1 à 30$\mu$m, et un diamètre moyen en volume allant de 5 à 150$\mu$m. Ces particules peuvent être en particulier obtenues par le procédé tel que décrit dans le brevet EP1917098B1 commenté précédemment.

Les particules selon la présente invention permettent, au sein des compositions aérosols anhydres, d'encapsuler des ingrédients particulièrement fragiles et volatiles en conservant l'exacte composition mise au point par le formulateur. Elles sont étanches en l'absence de stimuli et notamment restent inodores pour les ingrédients encapsulés odorants tels que les ingrédients de parfumerie. Lesdites particules sont stables au sein des compositions aérosols anhydres et peuvent libérer tout ou partie de leur contenu quasi-instantanément au contact d'eau et tout particulièrement de l'humidité atmosphérique ou de la transpiration. Grâce à leur densité de poudre versée (densité apparente non tassée) faible, elles ne sédimentent pas ou du moins peu. Elles sont compatibles avec les gaz propulseurs et résistantes à la compression pour pouvoir être formulées dans un dispositif aérosol sans être endommagées.

Cette découverte est à la base de la présente invention.

La présente invention concerne une composition anhydre comprenant :

1) au moins des particules comprenant un cœur contenant au moins un agent bénéfique et une enveloppe entourant le coeur; ladite enveloppe comprenant au moins un polysaccharide modifié hydrophobe choisi parmi les $C_5$-$C_{20}$-alcényl succinate d'amidon et au moins un glucide hydrosoluble et/ou un polyol hydrosoluble choisi parmi les maltodextrines de DE allant de 4 à 20;
lesdites particules présentant simultanément une densité de poudre versée allant de 300,0g/l à 600,0g/l et une densité absolue supérieure à 1,0, les particules étant sphériques, et avant un diamètre moyen en nombre allant de 1 à 30$\mu$m, et un diamètre moyen en volume allant de 5 à 150$\mu$m et
2) au moins un agent propulseur.

De préférence, la composition comprend un milieu physiologiquement acceptable.

Selon une forme particulière de l'invention, les compositions sont cosmétiques ou dermatologiques.

Selon une forme particulière de l'invention, les compositions selon l'invention peuvent être utilisées dans d'autres applications industrielles et notamment être des produits de consommation choisis parmi des produits vétérinaires; des produits d'hygiène et/ou de soin des animaux; des produits d'entretien ménager comme les produits pour le soin et/ou le nettoyage du linge (détachants, adoucissants, produits pour le repassage), les produits de nettoyage et/ou d'entretien des appareils électroménagers, les produits de nettoyage et/ou d'entretien des sols, carrelages, bois etc ; des produits sanitaires comme des désodorisants, des produits détartrants, des produits déboucheurs de canalisations ; des produits d'entretien des matières textiles, des produits d'entretien pour la maroquinerie comme les chaussures, les semelles; des produits issus de l'industrie agro-alimentaire; des produits issus de l'agriculture; des produits phytosanitaires; des peintures; des encres; des produits d'entretien dans l'industrie automobile.

L'invention concerne également un procédé cosmétique de soin et/ou d'hygiène et/ou de parfumage et/ou de maquillage d'une matière kératinique consistant à appliquer sur ladite matière kératinique une composition telle que définie précédemment.

L'invention concerne encore un procédé de traitement cosmétique des odeurs corporelles et éventuellement de la transpiration humaine consistant à appliquer sur une matière kératinique une composition telle que définie précédemment comprenant au moins un actif déodorant et/ou un actif anti-transpirant sous forme libre et/ou sous forme encapsulée.

L'invention concerne également un dispositif aérosol constitué par un récipient comprenant une composition aérosol telle que définie précédemment et par un moyen de distribution de ladite composition.

L'invention concerne aussi un produit de consommation, caractérisé par le fait qu'il est conditionné dans un dispositif aérosol constitué par un récipient comprenant une composition telle que définie précédemment et par un moyen de distribution de ladite composition.

Définitions

Par «composition anhydre», on entend au sens de la présente invention, une composition présentant une teneur en eau inférieure à 5 % en poids, de préférence inférieure à 2% en poids et de manière encore plus préférée inférieure à 1% en poids par rapport au poids de ladite composition voire encore moins de 0,5% et notamment exempte d'eau. Dans cette définition, l'eau mentionnée inclut l'eau résiduelle apportée par les ingrédients mélangés

Au sens de la présente invention, on entend désigner par «milieu physiologiquement acceptable», un milieu convenant à l'administration d'une composition par voie topique. Un milieu physiologiquement acceptable est généralement un milieu sans odeur désagréable et/ou sans aspect désagréable, et qui est parfaitement compatible avec la voie d'administration topique.

Par "matière kératinique", on entend la peau, le cuir chevelu, les lèvres, et/ou les phanères tels que les ongles et les fibres kératiniques, telles que par exemple, les poils, les cils, les sourcils et les cheveux.

Par «composition cosmétique», on entend au sens de l'invention toute composition appliquée sur une matière kératinique pour produire un effet non-thérapeutique d'hygiène, de soin, de conditionnement ou de maquillage contribuant à l'amélioration du bien-être et/ou à l'embellissement et/ou la modification de l'aspect de la matière kératinique sur laquelle on applique ladite composition.

Par «composition dermatologique», on entend au sens de l'invention toute composition appliquée sur une matière kératinique pour prévenir et/ou traiter un désordre ou un dysfonctionnement de ladite matière kératinique.

Par «traitement cosmétique», on entend au sens de l'invention tout effet non-thérapeutique de parfumage, d'hygiène, de soin, de conditionnement ou de maquillage contribuant à l'amélioration du bien-être et/ou à l'embellissement et/ou la modification de l'aspect ou de l'odeur de la matière kératinique sur laquelle on applique ladite composition.

Par «produit de consommation», on entend tout produit fabriqué destiné à être utilisé ou consommé dans la forme où il est commercialisé et qui n'est pas destiné à une fabrication ou modification ultérieure. Sans que les exemples soient limitatifs, les produits de consommation selon l'invention peuvent être des produits cosmétiques incluant aussi bien des formulations cosmétiques de soin et/ou d'hygiène et/ou de maquillage de la peau, des lèvres, des ongles, des cils, des sourcils, des cheveux ou du cuir chevelu ; des produits pour l'hygiène buccale tels que des désodorisants pour l'haleine ; des produits dermatologiques; des produits de parfumerie; des produits pharmaceutiques; des produits à usage vétérinaire notamment des produits d'hygiène et/ou le soin des animaux; des produits d'entretien ménager comme des produits pour le soin et/ou le nettoyage du linge (détachants, adoucissants, produits pour le repassage); des produits de nettoyage et/ou d'entretien des appareils électroménagers; des produits de nettoyage et/ou d'entretien des sols, carrelages, bois etc ; des produits sanitaires comme des désodorisants, des produits détartrants, des produits déboucheurs de canalisations; des produits d'entretien des matières textiles; des produits d'entretien dans la maroquinerie comme les chaussures, les semelles; des produits issus de l'industrie agro-alimentaire; des produits issus de l'agriculture; des produits phytosanitaires; des peintures; des encres; des produits d'entretien dans l'industrie automobile.

Par «agent bénéfique», on entend au sens de l'invention tout composé présent dans un produit de consommation produisant un effet bénéfique perçu par le consommateur lors de son utilisation et/ou obtenu sur le produit de consommation lui-même, ledit effet bénéfique pouvant être une amélioration sensorielle ou une modification notamment visuelle et/ou olfactive et/ou tactile, une amélioration du confort et /ou de la facilité d'application, un effet esthétique, un effet hygiénique, une sensation de propreté, un effet curatif et/ou prophylactique.

Par «particules comprenant un cœur contenant au moins un agent bénéfique», on entend une particule comprenant au moins un agent bénéfique immobilisé, capturé et/ou encapsulé dans la matrice d'un système d'encapsulation ou de piégeage; ledit agent bénéfique étant libéré vers l'extérieur au fur et à mesure de la détérioration du système d'encapsulation ou de piégeage lorsque sa dégradation se produit au contact d'un milieu avec lequel il va réagir ou sous l'effet d'un stimulus tel qu'un apport d'eau.

**Densité de poudre versée (ou densité apparente non tassée)**

La détermination est effectuée à température ambiante (20-25°C) et dans les conditions normales atmosphériques (1 atmosphère) à l'aide d'une éprouvette de 100ml. L'éprouvette est pesée vide puis remplie d'un volume de 100ml de poudre versée, sans tassement. La différence de masse entre éprouvette vide et remplie de 100ml de poudre donne la densité de poudre versée.

**Densité absolue**

Principe de la mesure

La mesure consiste à déterminer le poids d'un échantillon du solide en poudre par simple pesée puis à mesurer le volume occupé par les particules de poudre en mesurant le volume de liquide déplacé par l'échantillon de poudre par immersion dans ce liquide. Le liquide choisi doit être peu volatil et ne doit pas être un solvant de la poudre. On choisit généralement le cyclohexane. Les mesures sont réalisées au moins deux fois.

Matériels : Une fiole jaugée de 10 ou 25ml et une balance de précision.

- $m_1$ est le poids de la fiole vide

- $m_2$ est le poids de la fiole remplie d'eau jusqu'au trait de jauge.

- $m_3$ est le poids de la fiole remplie de cyclohexane jusqu'au trait de jauge.

- $m_4$ est le poids de la fiole remplie environ au tiers de sa capacité par la poudre à analyser.

On remplit la fiole environ au tiers de sa capacité avec la poudre à analyser.

Méthode

On complète la fiole, un peu en-dessous du trait de jauge avec du cyclohexane. Afin d'éliminer complètement l'air emprisonné dans la poudre on opère comme suit :

1) on traite la fiole dans un bac à ultra-sons pendant 5 minutes
2) on ajuste le niveau du cyclohexane jusqu'au trait de jauge
3) on traite la fiole dans un bac à ultra-sons pendant 2 minutes
4) les étapes 2 et 3 sont répétées si nécessaire jusqu'à ce que le niveau du cyclohexane n'évolue plus.

$m_5$ est le poids de la fiole ainsi remplie.

Le poids de poudre analysée est égal à $m_4 - m_1$ (pour une bonne précision ce poids doit être supérieur à 2g). La densité de l'air étant très faible par rapport à celle du solide on admet que $m_4 - m_1$ est égal au poids du solide constitutif de la poudre.

Le poids de cyclohexane correspondant au volume occupé par le solide (Vs) est égal à :
$m_6 = (m_3 - m_1) - (m_5 - m_4) = \rho_{cyclo} \cdot Vs$ où $\rho_{cyclo}$ est la densité du cyclohexane à la température du laboratoire.

La densité absolue du solide constitutif de la poudre est égale à

$$\rho_{cyclos} = (m_4 - m_1) / Vs = \rho_{cyclo}(m_4 - m_1) / m_6.$$

Si la densité du cyclohexane à la température du laboratoire n'est pas connue, on la détermine comme suit par rapport à celle de l'eau :
Soit Vf le volume jaugé de la fiole et $\rho_{eau}$ la densité de l'eau à la température du laboratoire on a :

$$\rho_{cyclo} = (m_3 - m_1) / Vf \text{ et } \rho_{eau} = (m_2 - m_1) / Vf$$

soit

$$\rho_{cyclo} = \rho_{eau} (m_2 - m_1) / (m_3 - m_1)$$

La densité absolue du solide constitutif de la poudre est égale à :

$$\rho_s = [\rho_{eau} (m_4 - m_1) (m_2 - m_1)] / [m_6 (m_3 - m_1)].$$

## PARTICULES D'ENCAPSULATION

Les particules conformes à l'invention comprennent un cœur contenant au moins un agent bénéfique et une enveloppe entourant le cœur; ladite enveloppe comprenant au moins un polysaccharide modifié hydrophobe choisi parmi les $C_5$-$C_{20}$-alcényl succinate d'amidon et au moins un glucide hydrosoluble choisi parmi les maltodextrines de D.E. allant de 4 à 20; lesdites particules présentant simultanément une densité de poudre versée allant de 300,0g/l à 600,0g/l et une densité absolue supérieure à 1,0; les particules étant sphériques, et ayant un diamètre moyen en nombre allant de 1 à 30$\mu$m, et un diamètre moyen en volume allant de 5 à 150$\mu$m.

Les particules conformes à la présente invention sont de préférence sphériques.

Par «sphériques», on entend que la particule présente un indice de sphéricité, c'est-à-dire le rapport entre son plus grand diamètre et son plus petit diamètre, est inférieur à 1,2. Dans ce cas, de telles particules sont généralement appelées «capsules».

Par « taille moyenne » des particules on entend les paramètres D[4,3] et D[2,3] mesurés en voie sèche par diffraction laser à l'aide d'un granulomètre Microtrac S3500, les résultats étant exprimés sous la forme de distributions granulométriques en volume et en nombre donnant accès au diamètre moyen.

Les particules sphériques conformes à la présente invention présentent, de préférence, ainsi un diamètre moyen en nombre allant de 1 à 30$\mu$m plus préférentiellement allant de 2 à 15$\mu$m et encore mieux de 5 à 10$\mu$m et un diamètre moyen en volume allant de 5 à 150$\mu$m, de préférence allant de 10 à 100$\mu$m et encore mieux de 20 à 80$\mu$m.

Les particules selon l'invention contenant l'agent bénéfique représentent, de préférence, de 0,1 à 60% en poids, de préférence 0,3 à 40% en poids et encore mieux de 0,5 à 20% en poids du poids total de la composition.

## POLYSACCHARIDE MODIFIE HYDROPHOBE

On entend par «polysaccharide modifié hydrophobe» tout polysaccharide modifié par voie chimique ou enzymatique et comportant au moins un groupe fonctionnel hydrophobe.

Les polysaccharides sont des macromolécules glucidiques formées par l'enchaînement d'un grand nombre de sucres élémentaires (oses) hydrophiles liés entre eux par des liaisons O-osidiques.

Les groupes fonctionnels hydrophobes de la présente invention sont des groupes hydrocarbonés (constitués essentiellement d'atomes de carbone et d'hydrogène) comprenant de préférence au moins 6 et encore mieux au moins 8 atomes de carbone alcènyles. Le nombre maximum d'atomes de carbone du groupe hydrocarboné est, de préférence, 24, plus préférentiellement 20, et encore plus préférentiellement 18. Les groupes hydrocarbonés hydrophobes peuvent être non substitués, par exemple constitués d'une simple longue chaine alkyle ou bien substitués par des groupes non réactifs comme des groupes aromatiques tels que des groupes aryles (ie: phényle) ou aralkyles (ie: benzyle) ou encore des groupes polaires tels que par exemple des carboxyles ou des hydroxyles.

Pour greffer le ou les groupe(s) fonctionnel(s) hydrophobe(s) sur les polysaccharides, on utilise généralement des dérivés des acides carboxyliques ou leurs dérivés (esters, halogénures d'acides, anhydrides)

Le polysaccharide modifié hydrophobe représente de préférence de 20 à 90% en poids, notamment 30 à 80% en poids, mieux 40 à 70% en poids, et encore mieux 40 à 60% en poids par rapport au poids total de l'enveloppe de la particule.

Les molécules d'amidons peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

On entend par «amidon modifié hydrophobe» tout amidon modifié par réaction chimique ou enzymatique et comprenant au moins un groupe fonctionnel hydrophobe.

Les amidons modifiés hydrophobes conformes à l'invention sont choisis parmi les $C_5$-$C_{20}$ alcényl succinates d'amidon, plus particulièrement l'octényle succinate d'amidon sodique (E1450- CAS 66829-29-6 / 52906-93-1 / 70714-61-3), en particulier celui commercialisé par National Starch sous la dénomination CAPSUL®.

On peut également citer les références commerciales CAPSUL TA®, N-LOK®, N- LOK 1930®, HI-CAP 100®, PURITY GUM 1773®, PURITY GUM 2000 ® de National Starch, CLEARGUM CO® de la société Roquette et EMCAP®, EMTEX®, DELITEX de la société Cargill.

## GLUCIDE HYDROSOLUBLE

Par «glucide hydrosoluble », on entend un glucide ou polyol qui, introduit dans l'eau sans modification de pH à 25°C, à une concentration massique égale à 3%, permet l'obtention d'une solution macroscopiquement homogène et transparente, c'est à dire ayant une valeur de transmittance minimum de la lumière, à une longueur d'onde égale à 500nm, à travers un échantillon de 1cm d'épaisseur, d'au moins 80%, de préférence d'au moins 90%.

Par «glucides» (encore appelés hydrates de carbone ou carbohydrates ou saccharides) on entend l'ensemble des sucres simples ou oses et leurs combinaisons ou osides.

Les glucides comprennent habituellement :

(1) les monosaccharides ou oses qui sont de deux types : les aldoses comprenant une fonction aldéhyde sur le premier carbone et les cétoses comprenant une fonction cétone sur le deuxième carbone. On les distingue aussi suivant le nombre d'atomes de carbone qu'ils possèdent.

(2) les oligosaccharides (ou oligosides) qui sont des oligomères d'oses ayant un enchaînement de 2 à 10 unités monosaccharides unies par des liaisons glycosidiques.

(3) Les polyholosides (ou polysaccharides ou polyosides) qui sont des polymères d'oses ayant un enchaînement de monosaccharides supérieur à 10 unités

## GLUCIDES HYDROSOLUBLES

Les glucides hydrosolubles de l'invention sont choisi parmi les maltodextrines les maltodextrines ayant un Dextrose Equivalent allant de 4 à 20.

Les maltodextrines qui sont le résultat de l'hydrolyse d'un amidon (ie : blé, maïs) ou d'une fécule (ie : pomme de terre). Elles sont constituées de différents sucres (ie : glucose, maltose, maltotriose, oligosides et polyosides) directement issus de cette réaction, dans des proportions qui dépendent du degré de l'hydrolyse.

Ce degré est mesuré par « dextrose équivalent », ou D.E., le dextrose ou D-glucose étant le résultat d'une hydrolyse totale de l'amidon. Plus le D.E. est élevé, plus l'hydrolyse est poussée, et donc plus la proportion en sucres simples (à chaîne courte) composant la maltodextrine est élevée.

Les maltodextrines utilisées conformément à l'invention sont préférentiellement de D.E. allant de 12 à 20.

On utilisera de préférence les maltodextrines de pomme de terre ou de maïs telles que celles vendues sous les dénominations commerciales MD 20P® de AVEBE, MALDEX 120®, MALDEX 170®, MALDEX 190® de Tereos.

Parmi les glucides hydrosolubles conformes à l'invention, on choisi les maltodextrines de D.E. allant de 4 à 20 et encore mieux les maltodextrines de D.E. allant de 12 à 20.

Le ou les glucides hydrosoluble(s) conformes à l'invention représentent de 10 à 80% en poids, de préférence de 15 à 70% en poids, plus préférentiellement de 20 à 65% en poids, et encore mieux de 40 à 60% en poids par rapport au poids total de l'enveloppe de la particule.

Selon l'invention, l'enveloppe des particules selon l'invention est constituée

- d'au moins un $C_5$-$C_{20}$-alcényl succinate d'amidon et
- d'au moins une maltodextrine de D.E. allant de 4 à 20 et de préférence allant de 12 à 20.

Selon une forme particulièrement préférée de l'invention, l'enveloppe des particules d'encapsulation est constituée :

a) d'au moins un $C_5$-$C_{20}$-alcényl succinate d'amidon dans une quantité allant de 20 à 90% en poids, notamment 30 à 80% en poids, de préférence 40 à 70% et encore mieux 40 à 60% en poids, par rapport au poids total de l'enveloppe de la particule.

b) et d'au moins une maltodextrine de D.E. allant de 4 à 20 dans une quantité allant de 10 à 80% en poids, de préférence de 15 à 70% en poids, plus préférentiellement de 20 à 65% en poids, et encore mieux de 40 à 60% en poids par rapport au poids total de l'enveloppe de la particule.

## Procédé de préparation des particules d'encapsulation

Les particules selon l'invention peuvent notamment être préparées selon le procédé décrit dans le brevet EP1917098B1 de FeyeCon.

Selon une forme particulière de l'invention, les particules sont obtenues selon un procédé comprenant au moins les étapes suivantes :

- on prépare une solution aqueuse constituée du mélange du glucide hydrosoluble choisi parmi les maltodextrines de Dextrose Equivalent allant de 4 à 20 et du polysaccharide modifié hydrophobe choisi parmi les $C_5$-$C_{20}$-alcényl succinate d'amidon puis on ajoute l'agent bénéfique et on agite de façon à former une émulsion; et
- on homogénéise ladite émulsion ainsi formée sous haute pression à une pression allant de 10 à 200bars et plus préférentiellement de 20 à 200bars; et

- on pulvérise, de préférence en continu, ladite émulsion dans une chambre de séchage ; et
- l'eau est extraite pendant une durée, de préférence, ne dépassant pas 3 heures, et plus préférentiellement ne dépassant pas 30 minutes, avec un fluide sous pression tel que le dioxyde de carbone, de préférence à l'état supercritique, de préférence à une pression d'au moins 0,3XPc et à une température d'au moins Tc-60°C avec Pc correspondant à la pression critique du gaz et Tc la température critique du gaz, de façon à obtenir des particules, de préférence sphériques, de taille moyenne, de préférence allant de 1 à 150$\mu$m, plus préférentiellement allant de 2 à 100$\mu$m et encore mieux de 5 à 80$\mu$m.

## AGENT PROPULSEUR

Comme indiqué précédemment, la composition comprend un ou plusieurs agents propulseurs.

L'agent propulseur utilisé dans la composition cosmétique selon l'invention est choisi parmi le diméthyléther, les hydrocarbures volatils tels que le propane, l'isopropane, le n-butane, l'isobutane, le n-pentane et l'isopentane et leurs mélanges, éventuellement avec au moins un hydrocarbure chloré et/ou fluoré; parmi ces derniers on peut citer les composés vendus par la société Dupont de Nemours sous les dénominations FREON® et DYMEI®, et en particulier le monofluorotrichlorométhane, le difluorodichlorométhane, le tétrafluorodichloroéthane et le 1,1-difluoroéthane vendu notamment sous la dénomination commerciale DYMEL 152 A® par la société DUPONT.

On peut également utiliser en tant qu'agent propulseur le gaz carbonique, le protoxyde d'azote, l'azote ou l'air comprimé.

De préférence, la composition selon l'invention comprend un agent propulseur choisi parmi les hydrocarbures volatils.

Plus préférentiellement, l'agent propulseur est choisi parmi le diméthyléther, le propane, l'isopropane, le n-butane, l'isobutane, le pentane et l'isopentane et leurs mélanges.

Le rapport pondéral entre la composition sans propulseur (jus) et l'agent propulseur varie de préférence dans un rapport de 5/95 à 60/40, de préférence de 10/90 à 50/50, et plus préférentiellement de 15/85 à 30/70.

Un autre objet de la présente invention est un dispositif aérosol constitué par un récipient comprenant une composition telle que définie précédemment et par un moyen de distribution de ladite composition.

Le moyen de distribution, qui forme une partie du dispositif aérosol, est généralement constitué par une valve de distribution commandée par une tête de distribution, elle-même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

Les dispositifs conformes à l'invention sont bien connus de l'homme de l'art et comprennent les récipients aérosols comprenant un propulseur ainsi que les pompes aérosols utilisant l'air comprimé comme propulseur. Ces derniers sont décrits dans les brevets US 4,077,441 et US 4,850,517.

## AGENTS BENEFIQUES

La quantité d'agent bénéfique présent dans les particules conformes à l'invention varie de préférence de 0,1 à 80% en poids du poids de la particule, de préférence 1 à 70% en poids, mieux de 10 à 60% et encore mieux de 15 à 50% en poids du poids total de la particule.

Le temps de libération de l'agent bénéfique variera évidemment selon la nature et l'intensité du stimulus.

La durée totale pour libérer l'agent bénéfique pourra être modulée et sera fortement dépendante de la composition de la formule aérosol, de la teneur en particules présentes dans l'aérosol, de la nature notamment chimique de l'agent bénéfique et de sa concentration dans les particules (quantité encapsulée dans la particule) et de la nature et de l'intensité du stimulus auquel sera soumise la particule contenant l'agent bénéfique. La libération peut aussi bien être quasi instantanée ou durer plusieurs heures voire plusieurs jours.

Parmi les agents bénéfiques utilisables selon l'invention, on peut citer plus particulièrement

(i) les corps gras;
(ii) les substances parfumantes;
(iii) les principes actifs pharmaceutiques;
(iv) les actifs cosmétiques.

### Corps gras

Les corps gras sont couramment utilisés dans la formulation de. Ils peuvent être choisis dans le groupe comprenant

(i) les huiles naturelles d'origine végétale, animale ou marine
(ii) les huiles minérales,
(iii) les huiles hydrogénées,
(iv) les huiles de silicone,
(v) les terpènes,
(vi) le squalène,
(vii) les acides gras saturés ou insaturés
(viii), les esters d'acide gras,
(x) les cires,
(x) les alcools gras,
(xi) les beurres comme le beurre de karité ou le beurre de cacao,
(xii) et leurs mélanges

**Les substances parfumantes**

Par «substance parfumante», on entend tout ingrédient susceptible de dégager une odeur agréable.

Les parfums sont des compositions contenant notamment des matières premières (dénommées généralement ingrédients de parfumerie) qui sont décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits de synthèse ou de produits naturels, comme des huiles essentielles, des absolus, des résinoïdes, des résines, des concrètes, et/ou des produits synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage. L'huile essentielle est le plus souvent séparée de la phase aqueuse par un procédé physique n'entraînant pas de changement significatif de la composition.

Parmi les huiles essentielles utilisables selon l'invention, on peut citer celles obtenues à partir des plantes appartenant aux familles botaniques suivantes :
Abiétaceés ou Pinacées : conifères; Amaryllidacées; Anacardiacées; Anonacées : ylang; Apiacées (par exemple les ombellifères) : aneth, angélique, coriandre, criste marine, carotte, persil; Aracées; Aristolochiacées; Astéracées : achilée, armoise, camomille, hélichryse; Bétulacées; Brassicacées; Burséracées : encen ; Caryophyllacées; Canellacées; Césalpiniacées : copaïfera (copahu); Chénopodacées; Cistacées : ciste; Cypéracées; Diptérocarpacées; Ericacées : gaulthérie (wintergreen); Euphorbiacées; Fabacées; Geraniacées : géranium; Guttifères; Hamamélidacées; Hernandiacées; Hypéricacées : millepertuis; Iridacées; Juglandacées; Lamiacées : thym, origan, monarde, sarriette, basilic, marjolaines, menthes, patchouli, lavandes, sauges, cataire, romarin, hysope, mélisse; Lauracées : ravensara, laurier, bois de rose, cannelle, litséa; Liliacées : ail, lys, muguet, jacinthe, jonquille,...; Magnoliacées : magnolia; Malvacées ; Méliacées; Monimiacées; Moracées : chanvre, houblon; Myricacées; Mysristicacées : muscade; Myrtacées: eucalyptus, tea tree, niaouli, cajeput, backousia, girofle, myrte; Oléacées; Pipéracées : poivre ; Pittosporacées; Poacées : citronnelle, lemongrass, vétiver; Polygonacées; Renonculacées; Rosacées : roses; Rubiacées; Rutacées : tous les citrus; Salicacées; Santalacées : santal; Saxifragacées; Schisandracées; Styracacées : benjoin; Thymélacées : bois d'agar; Tilliacées; Valérianacées : valériane, nard; Verbénacées : lantana, verveine; Violacées; Zingibéracées : galanga, curcuma, cardamome, gingembre; Zygophyllacées.

On peut citer également les huiles essentielles extraites de fleurs (lys, lavande, rose, jasmin, Ylang-Ylang, néroli), de tiges et de feuilles (patchouli, géranium, petit-grain), de fruits (framboise, pêche ,coriandre, anis, cumin, genièvre), d'écorces de fruits (bergamote, citron, orange, pamplemousse), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes (galbanum, élémi, benjoin, myrrhe, oliban, opopanax).

Des exemples de substances parfumantes sont notamment : le géraniol, l'acétate de géranyle, le farnésol, le bornéol, l'acétate de bornyle, le linalool, l'acétate de linalyle, le propionate de linalyle, le butyrate de linalyle, le tétrahydrolinalool, le citronellol, l'acétate de citronellyle, le formiate de citronellyle, le propionate de citronellyle, le dihydromyrcénol, l'acétate de dihydromyrcényle, le tétrahydromyrcénol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzylcarbinol, l'acétate de trichlorométhylphénylcarbinyle, l'acétate de p-tert-butylcyclohexyle, l'acétate d'isononyle, l'acétate de cis-3-hexenyle , l'acétate de vétivéryle, l'acétate d'éthyle, l'acétate de butyle, l'acétate d'hexyle, l'acétate de décyle, l'acétate d'isoamyle, l'acétate de stéaryle, l'heptanoate d'allyle, le vétivérol, l'alpha-hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, l'allyl 3-cyclohexylpropionate, l'éthyl-6-(acétyloxy)-hexanoate, le caproate d'allyle, l'éthyl-2 méthyl butyrate, le méthyl dihydrojasmonate, le salicylate d'hexyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, l'anisyl propanal, le 9-décènol-1, le cis-3-hexénol, le tétrahydro-2-isobutyl-4-méthylpyrann-4-ol, 3-Methyl-5-phényl-1-pentanol, le 3a,6,6,9a-Tétraméthyl-dodécahydronaphtho[2,1-b]furanne, l'isobutyrate de phénoxyéthyle, le phényl-acétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, l' hexylcinnamal, le 2,4-dimethylcyclohex-3-ène-1-carbaldéhyde, le 2,6-dimethylhept-5-ènal, l'$\alpha,\alpha$-di-méthyl-p-éthylphénylpropanal, le 1,3-benzodioxole-5-carboxaldéhyde, le limonène, la damascone, la décalactone, la nonalactone, le 6,6-diméthoxy-2,5,5-triméthylhex-2-ène, la 2,4,4,7-tétraméthyloct-6-èn-3-one, la 1-(5,5-diméthyl-1-cyclohexenyl)-pent-4-èn-1-one, la méthylheptènone, le 4-(cyclopropylméthyl)-phénylméthyl éther, le 2-methyl-6-méthyli-deneoct-7-en-2-ol, l'oxyde de rose, la 1-(1,2,3,4,5,6,7,8-octahydro-2,3,8,8-tetraméethyl-2-naphthyl)éthan-1-one, la 2-acétonaphthone, la 2-isopropyl-5-méthylcyclohexanone, les ionones, les méthylionones, les isométhylionones, la solanone, les irones, le cis-3-hexénol et ses esters, les muscs-indanes, les muscs-tétralines, les muscs-isochromanes, les cétones macrocycliques, les muscs-macrolactones, les muscs aliphatiques, le brassylate d'éthylène, l'essence de rose et leurs mélanges.

D'une façon générale, les parfums sont constitués d'un mélange d'ingrédients dits de parfumerie qui peuvent être également classés en notes de tête, notes de cœur et notes de fond.

Les trois notes correspondent à la volatilité plus ou moins importante des ingrédients qui les composent : note de tête fortement volatile, note de coeur moyennement volatile et note de fond faiblement volatile.

(i) La note de tête appelée aussi « départ » est celle que l'odorat perçoit en premier dès que le parfum est en contact avec la matière kératinique ou tout substrat. Mais c'est celle qui s'atténue le plus rapidement : elle ne "tient pas". Il est difficile d'exprimer le temps de persistance de cette note, car il est très variable : de quelques minutes à une dizaine de minutes
Elle est essentiellement fraîche et légère. Tous les agrumes appartiennent notamment à cette catégorie. En parfumerie, on les range sous le terme générique d'hespéridés dont font partie orange, citron, pamplemousse, bergamote, néroli etc... On citera également les aromates tels que la lavande, le laurier, le thym ou le romarin et les anisés, mentholés, aldéhydés, etc. On citera également les notes eucalyptus.

(ii) La note de cœur, parfois appelée aussi «corps» a une persistance qui va de quelques dizaines de minutes à quelques heures, mais sa principale caractéristique est de ne se révéler qu'au bout de quelques minutes. Elle "démarre" donc juste avant l'extinction de la note de tête. Elle commence à s'exprimer alors que la note de tête s'efface progressivement. Elle est représentée essentiellement par des éléments floraux, fruités ou épicés : muguet, chèvrefeuille, violette, magnolia, cannelle, géranium, jasmin, rose, iris, framboise, pêche, etc...

(ii) La note de fond, parfois appelée aussi «fond» assure la "durabilité", la persistance ou la ténacité d'un parfum. Elle est perceptible plusieurs heures, voire plusieurs jours, ou même plusieurs semaines après application. sur un vêtement ou sur une mouillette ou touche olfactive, selon la concentration du parfum. On citera par exemple les bois, racines, mousses, résines et les substances animales ou minérales telles que opoponax, muscs, ambre, santal, benjoin, lichen, clou de girofle, sauge, etc. On citera également les notes vanillées, le patchouli, les coumarines...etc.

On peut bien entendu encapsuler des ingrédients appartenant à une ou plusieurs notes. Toutefois, on préférera encapsuler les ingrédients les plus volatiles (= les moins rémanents) appartenant aux notes de tête et/ou de cœur. Parmi ces ingrédients, on citera, par exemple :

Acétate de benzyle
Acétate de géranyle
Acétate de cis-3-hexenyle
Aldéhyde C18 ou nonalactone
Acétate de décyle

Allyl amyl glycolate (citral)
Acétate d'éthyle
Acétate de butyle
Allyl 3-cyclohexylpropionate
Acétate de linalyle
Alcool Phényléthylique
Acétate d'héxyle
Berryflor ou éthyl-6-(acétyloxy)-hexanoate
Acétate d'isoamyle
Caproate d'allyle
Amarocite ou 6,6-diméthoxy-2,5,5-triméthylhex-2-ène
Citral lemarome N ou 3,7-diméthylocta-2,6-diénal
Canthoxal ou anisyl propanal
Claritone ou 2,4,4,7-tétraméthyloct-6-èn-3-one
Ethyl-2méthyl butyrate
Dihydromyrcénol
Cis-3 hexenol
Hédione ou méthyl dihydrojasmonate
L-carvone
Heptanoate d'allyle
Limonène
Néobuténone alpha ou 1-(5,5-diméthyl-1-cyclohexenyl)- pent-4-èn-1-one
Méthylheptènone
Toscanol ou 4-(cyclopropylméthyl)-phénylméthyl éther myrcenol super ou 2-methyl-6-methylideneoct-7-en-2-ol Decalactone
Acétate de stéaryle
Oxyde de rose
Linalool
Triplai ou 2,4-dimethylcyclohex-3-ène-1-carbaldéhyde Mélonal ou 2,6-dimethylhept-5-ènal
1-(1,2,3,4,5,6,7,8-octahydro-2,3,8,8-tetraméethyl-2-naphthyl)éthan-1-one
Hexylcinnamal
Tétrahydro-2-isobutyl-4-méthylpyrann-4-ol
Salicylate d'hexyle
1,4-Dioxacycloheptadécane-5,17-dione

et leurs mélanges

Selon une forme particulière de l'invention, les particules d'encapsulation comprennent au moins une ou plusieurs substances parfumantes ayant une pression de vapeur saturante à 25°C supérieure ou égale à 10,0 Pa

La pression de vapeur saturante (ou tension de vapeur) est la pression à laquelle la phase gazeuse d'une substance est en équilibre avec sa phase liquide ou solide à une température donnée dans un système fermé. Le calcul de la pression de vapeur saturante peut se faire à l'aide de la formule suivante :

$$\ln \frac{p_{\mathrm{sat}}}{p_0} = \frac{M.L_v}{R} \left( \frac{1}{T_0} - \frac{1}{T} \right)$$

avec :

- $T_0$: température d'ébullition de la substance à une pression $p_0$ donnée, en degrés Kelvin,
- $p_{sat}$: pression de vapeur saturante, dans la même unité que po
- M: masse molaire de la substance, en kg/mol
- $L_v$: chaleur latente de vaporisation de la substance, en Joules/kg
- R: constante des gaz parfaits, égale à 8,31447J/K/mol
- T: température de la vapeur, en K.

De préférence les substances parfumantes ayant une pression de vapeur saturante à 25°C supérieure ou égale à 10Pa représentent une quantité allant de 50 à 100% en poids, de préférence de 60 à 100% en poids, plus préférentiellement de 70 à 100% en poids, et encore mieux de 80 à 100% en poids par rapport au poids total des substances parfumantes présentes dans les particules de l'invention.

## Les principes actifs pharmaceutiques

On entend par «principe actif pharmaceutique» une molécule ou un mélange de molécules qui possède un effet thérapeutique, curatif et/ou prophylactique pouvant être administré par pulvérisation.

## Les actifs cosmétiques

On entend par «actif cosmétique» toute molécule qui possède un effet d'hygiène, de soin, de maquillage, de coloration contribuant à l'amélioration, du bien-être et/ou à l'embellissement ou la modification de l'aspect de la matière kératinique humaine sur laquelle on applique ladite composition.

Parmi les actifs cosmétiques susceptibles d'être appliqués sur des matières kératiniques humaines telles que la peau, les lèvres, le cuir chevelu, les cheveux, les cils ou les ongles, on peut citer par exemple, seuls ou en mélanges :

- les vitamines et leurs dérivés ou précurseurs, seuls ou en mélanges,
- les agents anti-oxydants;
- les agents nettoyants tels que les tensio-actifs;
- les matières colorantes;
- les agents conditionneurs;
- les agents pour le défrisage et/ou le lissage et/ou la mise en forme des cheveux;
- les agents anti-radicalaires;
- les agents photoprotecteurs comme les filtres UV organiques ou inorganiques;
- les agents autobronzants;
- les agents anti-glycation;
- les agents apaisants;
- les agents dépilatoires;
- les agents déodorants;
- les agents anti-transpirants;
- les inhibiteurs de NO-synthase;
- les agents stimulant la prolifération des fibroblastes;
- les agents stimulant la prolifération des kératinocytes;
- les agents dermorelaxants;
- les agents rafraîchissants;
- les agents tenseurs;
- les agents matifiants;
- les agents anti-luisance de la peau;
- les agents anti-séborrhéiques;
- les agents anti-cheveux gras;
- les agents dépigmentants;
- les agents pro-pigmentants;
- les agents kératolytiques;
- les agents desquamants;
- les agents hydratants;
- les agents anti-microbiens;
- les agents amincissants;
- les agents agissant sur le métabolisme énergétique des cellules;
- les agents répulsifs contre les insectes;
- les antagonistes de substances P ou de CRGP;
- les agents anti-chute des cheveux;
- les agents anti-rides;
- les agents anti-vieillissement;
- les agents antipelliculaires

Parmi ces actifs cosmétiques, on préférera tout particulièrement, seuls ou en mélanges

- les agents photoprotecteurs comme les filtres UV en particulier les filtres UV organiques;

- les agents anti-luisance de la peau;
- les agents anti-séborrhéiques;
- les agents anti-cheveux gras;
- les agents déodorants;
- les agents anti-transpirants;
- les agents rafraîchissants;
- les agents matifiants;
- les agents anti-microbiens;
- les agents antipelliculaires

Selon une forme particulièrement préférée de l'invention, le ou les agents bénéfiques présents dans les particules seront choisis parmi les substances parfumantes.

Selon une forme encore plus particulièrement préférée de l'invention, les substances parfumantes présentes dans les particules sont choisies parmi les notes de cœur et/ou les notes de tête de façon à pouvoir aussi bien compenser leur perte tout au long de la journée qu'à procurer un effet fraicheur supplémentaire au cours de la journée en réponse à la transpiration comme à l'humidité atmosphérique ou apportée par exemple par des brumisateurs.

Selon une forme particulière de l'invention, la composition contiendra des particules renfermant au moins une substance parfumante et au moins une substance parfumante sous forme libre, identique ou différente de la substance parfumante présente dans les dites particules.

Lesdites substances parfumantes sous forme libre peuvent être choisies parmi celles citées précédemment.

Selon une autre forme particulière de l'invention, la composition contient exclusivement la ou les substances parfumantes dans les particules d'encapsulation. Autrement dit, la totalité des ingrédients pour parfumer présents dans la composition sont contenus dans les particules.

La composition selon l'invention peut avantageusement comprendre au moins une phase grasse qui peut comprendre au moins un composé choisi parmi les huiles, les cires et/ou les solvants d'origine minérale, animale, végétale ou synthétique, carbonés, hydrocarbonés, fluorés et/ou siliconés, volatils ou non volatils, seuls ou en mélange dans la mesure où ils forment un mélange homogène et stable et sont compatibles avec l'utilisation envisagée.

Par 'volatil', on entend au sens de l'invention, tout composé susceptible de s'évaporer au contact des matières kératiniques en moins d'une heure, à température ambiante (25°C) et pression atmosphérique (1atm). Notamment, ce composé volatil a une pression de vapeur non nulle, à température ambiante et pression atmosphérique, notamment allant de 0,13Pa à 40 000Pa ($10^{-3}$ à 300mm de Hg), en particulier allant de 1,3Pa à 13 000Pa (0,01 à 100mm de Hg), et plus particulièrement allant de 1,3Pa à 1300Pa (0,01 à 10mm de Hg).

Par opposition, on entend par 'non volatil', un composé restant sur les matières kératiniques humaines à température ambiante et pression atmosphérique, au moins une heure et ayant notamment une pression de vapeur inférieure à $10^{-3}$mm de Hg (0,13Pa).

La composition peut comprendre, en outre, d'autres ingrédients sous forme libre (non encapsulés, emprisonnés dans les particules de l'invention) utilisés couramment dans les compositions cosmétiques. De tels ingrédients peuvent être choisis parmi les antioxydants, les conservateurs, les actifs cosmétiques tels que ceux cités précédemment, les substances parfumantes telles que celles décrites précédemment, les tensioactifs, les agents d'étalement, les agents mouillants, les agents dispersants, les anti-mousses, les neutralisants, les stabilisants, les polymères et notamment les polymères filmogènes liposolubles, et leurs mélanges.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition destinée à être conditionnée dans un dispositif aérosol.

L'homme du métier pourra choisir la composition appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Selon une forme particulière de l'invention, les compositions selon l'invention sont des produits de parfumage conditionnés dans un dispositif aérosol comme une eau de toilette, un parfum, un parfum d'ambiance dans lesquels les particules comprennent au moins une substance parfumante. Plus particulièrement, les compositions contiendront en plus une substance parfumante sous forme libre, identique ou différente de la substance parfumante présente dans les particules.

Selon une autre forme particulière de l'invention, les compositions selon l'invention peuvent se présenter sous forme de produit capillaire conditionné dans un dispositif aérosol. Ces produits capillaires peuvent être notamment des produits destinés au nettoyage non rincé des cheveux comme des «shampooings secs», destinés au coiffage et/ou à la mise en plis comme des laques, des mousses, des produits de conditionnement des cheveux : démêlants, brillants, lissants, etc..., des produits pour le soin et/ou le traitement cosmétique des cheveux et du cuir chevelu. Plus particulièrement, les particules comprennent au moins une substance parfumante.

Encore plus particulièrement, les compositions contiendront en plus au moins une substance parfumante sous forme libre, identique ou différente de la ou des substances parfumantes présentes dans les particules.

Selon une autre forme particulière de l'invention, les compositions selon l'invention peuvent se présenter sous forme de produit d'hygiène, en particulier des déodorants et/ou anti-transpirants où la composition comprend au moins un actif déodorant et/ou au moins un actif anti-transpirant, sous forme libre et/ou sous forme encapsulée. Plus particulièrement, les particules comprennent au moins une substance parfumante. Encore plus particulièrement, les compositions contiendront en plus au moins une substance parfumante sous forme libre, identique ou différente de la ou des substances parfumantes présentes dans les particules.

## Actif anti-transpirant

Par «actif anti-transpirant», on entend un composé qui, à lui seul, a pour effet de diminuer le flux sudoral, et/ou de diminuer la sensation d'humidité sur la peau liée à la sueur humaine et/ou d'absorber partiellement ou totalement la sueur humaine.

Parmi les actifs anti-transpirants, on peut citer les sels d'aluminium et/ou de zirconium tels que le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement le chlorhydrate d'aluminium sous forme activée ou non commercialisé par la société REHEIS sous la dénomination MICRODRY ALUMINUM CHLOROHYDRATE® ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF®, des sels d'aluminium « activés » par exemple celui commercialisé par la société REHEIS sous la dénomination REACH 103 ou par la société WESTWOOD sous la dénomination WESTCHLOR 200.

De préférence, la composition cosmétique comprend le chlorhydrate d'aluminium en tant qu'actif anti-transpirant.

Comme autre actif anti-transpirant, on peut citer les particules de perlite expansée telles que celles obtenues par le procédé d'expansion décrit dans le brevet US 5,002,698.

Les perlites utilisables selon l'invention sont généralement des aluminosilicates d'origine volcanique et ont comme composition

70,0-75,0% en poids de silice $SiO_2$
12,0-15,0% en poids d'oxyde d'aluminium oxyde $Al_2O_3$
3,0-5,0% d'oxyde de sodium $Na_2O$
3,0-5,0% d'oxyde de potassium $K_2O$
0,5-2% d'oxyde de fer $Fe_2O_3$ →
0,2-0,7% d'oxyde de magnesium $MgO$
0,5-1,5% d'oxyde de calcium $CaO$
0,05-0,15% d'oxyde de titane $TiO_2$

De préférence, les particules de perlite utilisées seront broyées; elles sont dans ce cas dites Expanded Milled Perlite (EMP). Elles ont de préférence une taille de particule définie par un diamètre médian D50 allant de 0,5 à 50 μm et de préférence de 0,5 à 40 μm.

De préférence, les particules de perlite utilisées présentent une densité apparente non tassée à 25°C allant de 10 et 400kg/m3 (Norme DIN 53468) et de préférence de 10 et 300kg/m3.

De préférence, les particules de perlite expansée selon l'invention ont une capacité d'absorption d'eau mesurée au WET POINT allant de 200 à 1500% et de préférence de 250 à 800%.

Le Wet Point correspond à la quantité d'eau qu'il faut additionner à 100 g de particules pour obtenir une pâte homogène. Cette méthode dérive directement de celle de la prise d'huile appliquée aux solvants. Les mesures sont faites de la même manière par l'intermédiaire du Wet Point et du Flow Point ayant respectivement comme définition suivante :

WET POINT : masse exprimée en grammes pour 100g de produit correspondant à l'obtention d'une pâte homogène lors de l'addition d'un solvant à une poudre.

FLOW POINT : masse exprimée en grammes pour 100g de produit à partir de laquelle la quantité de solvant est supérieure à la capacité de la poudre à le retenir. Cela se traduit par l'obtention d'un mélange plus ou moins homogène s'écoulant sur la plaque de verre.

Le Wet Point et le Flow point sont mesurés selon le protocole suivant :

Protocole de mesure de l'absorption d'eau.

1) Matériel utilisé

Plaque de verre (25 x 25mm)
Spatule (manche en bois et partie métallique (15 x 2,7mm)
Pinceau à poils de soie
Balance

2) Mode Opératoire

On dépose la plaque de verre sur la balance et on pèse 1g de particules de perlite. On dépose le bécher contenant le solvant ainsi que la liquipipette de prélèvement sur la balance. On ajoute progressivement le solvant à la poudre en malaxant régulièrement l'ensemble (toutes les 3 à 4 gouttes) à l'aide de la spatule
On note la masse de solvant nécessaire à l'obtention du Wet Point. On ajoute à nouveau le solvant et on note la masse permettant d'arriver au Flow Point. On effectuera la moyenne sur 3 essais.

On utilisera en particulier les particules de perlite expansée vendues sous les noms commerciaux OPTIMAT 1430 OR ou OPTIMAT 2550 par la société WORLD MINERALS.

**Actifs déodorants**

On appelle « actif déodorant », toute substance capable de masquer, absorber, améliorer et/ou réduire l'odeur désagréable résultant de la décomposition de la sueur humaine par des bactéries

Les actifs déodorants peuvent être des agents bactériostatiques ou des agents bactéricides agissant sur les germes des odeurs axillaires, comme le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltriméthylammonium, les sels de cétylpyridinium ; les polyols comme ceux de type glycérine, 1,3-propanediol (ZEMEA PROPANEDIOL® commercialisé par Dupont Tate and Lyle Bioproducts), le 1,2-décanediol (SYMCLARIOL® de la société Symrise) ; les dérivés de glycérine comme par exemple le Caprylic/Capric Glycerides (CAPMUL MCM® de Abitec), le Caprylate ou caprate de Glycerol (DERMOSOFT GMCY® et DERMOSOFT GMC® respectivement de STRAETMANS), le Polyglyceryl-2 Caprate (DERMOSOFT DGMC® de STRAETMANS) les dérivés de biguanide comme les sels de polyhexaméthylène biguanide ; la chlorhexidine et ses sels; le 4-Phenyl-4,4-dimethyl-2butanol (SYMDEO MPP® de Symrise) ; les cyclodextrines ; les chélatants tels que le Tetrasodium Glutamate Diacetate (CAS #51981-21-6) vendu sous le nom commercial DISSOLVINE GL-47-S® de Akzo Nobel, l'EDTA (acide Ethylendiamino Tétraacétique) et le DPTA (acide 1,3-diaminopropanetétraacétique).

Parmi les actifs déodorants conformes à l'invention, on peut aussi citer également

- les sels de zinc comme le salicylate de zinc, le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoléate de zinc, glycinate de zinc, carbonate de zinc, citrate de zinc, chlorure de zinc, le laurate de zinc, l'oléate de zinc, l'orthophosphate de zinc, le stéréate de zinc, le tartrate de zinc, l'acétate de zinc ou leurs mélanges ;
- des absorbeurs d'odeurs comme les zéolites notamment métalliques sans argent, les cyclodextrines, les silicates d'oxyde métallique telles que celles décrite dans la demande US2005/063928 ; des particules d'oxyde métallique modifiées par un métal de transition telles que décrites dans les demandes US2005084464 et US2005084474, des aluminosilicates comme ceux décrits dans la demande EP1658863, des particules de dérivés de chitosan comme celles décrites dans le brevet US6916465 ;
- le bicarbonate de sodium ;
- l'acide salicylique et ses dérivés tels que l'acide n-octanoyl-5-salicylique
- l'alun.

- le triéthyl citrate

Les actifs déodorants peuvent être présents de préférence dans les compositions selon l'invention dans des concentrations pondérales allant 0,01 à 10% en poids par rapport au poids total de la composition.

La présente invention concerne également un procédé cosmétique de traitement des odeurs corporelles et éventuellement de la transpiration humaine consistant à appliquer sur la matière kératinique une composition comprenant les particules telles que définies précédemment; ladite composition comprenant au moins un actif déodorant et/ou au moins un actif anti-transpirant, sous forme libre et/ou sous forme encapsulée.

L'invention est illustrée plus en détail dans les exemples suivants.

## Exemples de préparation de particules à libération de parfum Exemple A

On a préparé des capsules en mettant en œuvre la composition suivante :

| Composition | Amidon modifié hydrophobe | Polysaccharide hydrosoluble | Parfum* | Eau |
|---|---|---|---|---|
| **Exemple A** | Amidon Capsul® de National Starch 110g | Maltodextrine de pomme de terre MD 20 P de AVEBE 110g | 55g | 225g |

* Le parfum utilisé a la composition suivante :

| Ingrédients | Quantité en g |
|---|---|
| Myristate d'isopropyle | 20,5 |
| Méthyl dihydrojasmonate | 15 |
| 2-phenyléthanol | 8 |
| 1-(1,2,3,4,5,6,7,8-octahydro-2,3,8,8-tetraméthyl-2-naphthyl)éthan-1-one | 8 |
| Hexylcinnamal | 6 |
| Tétrahydro-2-isobutyl-4-méthylpyrann-4-ol | 6 |
| Hexyl Salicylate | 6 |
| Benzyl Acétate | 5 |
| 1,4-Dioxacycloheptadécane-5,17-dione | 5 |
| 3-Methyl-5-phényl-1-pentanol | 5 |
| dihydromyrcenol | 4 |
| ORANGE TERPENES 0,05% B H T (limonène >95%) | 4 |
| 2-acétonaphthone | 2 |

| | |
|---|---|
| 3a,6,6,9a-Tétraméthyl-dodécahydronaphtho[2,1-b]furanne | 1 |
| α,α-diméthyl-p-éthylphénylpropanal | 1 |
| 1,3-benzodioxole-5-carboxaldéhyde | 1 |
| 2-isopropyl-5-méthylcyclohexanone | 1 |
| 1-phényléthyl acetate | 0,8 |
| 2,6-dimethylhept-5-ènal (mélonal) | 0,5 |
| 2,4-dimethylcyclohex-3-ène-1-carbaldehyde (triplal) | 0,2 |

**Procédé de préparation de l'émulsion**

On a mélangé la maltodextrine de pomme de terre MD20 P et l'amidon CAPSUL® (sel de sodium de l'octényle succinate d'amidon) dans l'eau jusqu'à dissolution puis on a ajouté le parfum et émulsionné avec un disperseur Ultraturrax de type Heidolph Diax 900 (moteur de puissance 900W avec une vitesse de 8000 à 26000 tour/mn électroniquement contrôlée) à la puissance maximale pendant 4 minutes

**Procédure de séchage pour obtenir des particules sphériques**

L'émulsion obtenue a été ensuite homogénéisée à une pression de 30 bars au moyen d'une pompe à haute pression puis pulvérisée dans une chambre d'atomisation au moyen d'une buse simultanément avec un courant de $CO_2$ (30 bars, 45°C) que l'on a fait circuler en continu avec un débit d'environ 500g/mn pour éliminer l'eau. La poudre séchée a été retenue sur un filtre situé à la base de la chambre d'atomisation puis collectée après dépressurisation. On obtient ainsi 270g de microcapsules sphériques sous la forme d'une fine poudre blanche ayant un diamètre moyen en nombre de 7,8 $\mu$m et un diamètre moyen en volume de 47 $\mu$m.
La taille des particules a été mesurée en voie sèche par diffraction laser en utilisant un granulomètre Microtrac S3500, les granulométries étant exprimées en volume et en nombre.

| Exemple A | Caractéristiques mesurées des capsules | | | |
|---|---|---|---|---|
| | Taux de parfum encapsulé (%) | Taux de parfum libre (%) | Densité de poudre versée | Densité absolue |
| | 19,8 | < 0,1 | 484 | 1,12 |

**Exemples B à H**

Selon le procédé décrit à l'exemple A, on a préparé les capsules suivantes :

| Composition | Amidon modifié hydrophobe | Polysaccharide hydrosoluble | Parfum de l'exemple A | Eau |
|---|---|---|---|---|
| Exemple B | Amidon Capsul® de National Starch 110g | Maltodextrine MD 120 de Tereos 110g | 55g | 225g |
| Exemple C | Amidon Capsul® de National Starch 110g | Maltodextrine MD 170 de Tereos 110g | 55g | 225g |
| Exemple D | Amidon Capsul® de National Starch 110g | Maltodextrine MD 190 de Tereos 110g | 55g | 225g |
| Exemple E | Amidon Capsul® de National Starch 110g | Maltodextrine de pomme de terre MD 20 P de AVEBE 110g | 105g | 225g |
| Exemple F | Amidon Capsul® de National Starch 154g | Maltodextrine de pomme de terre MD 20 P de AVEBE 66g | 55g | 225g |
| Exemple G | Amidon Capsul® de National Starch 66g | Maltodextrine de pomme de terre MD 20 P de AVEBE 154g | 55g | 225g |
| Exemple H (hors invention) | Amidon Capsul® de National Starch 110g | Sirop de glucose Glucodry G290 de TEREOS 110g | 55g | 225g |

| Exemples | Caractéristiques mesurées des capsules | | | |
|---|---|---|---|---|
| | Taux de parfum encapsulé (%) | Taux de parfum libre (%) | Densité de poudre versée (g/l) | Densité absolue |
| Exemple B | 19,3 | <0,1 | 568 | 1,14 |
| Exemple C | 19,4 | <0,1 | 490 | 1,16 |
| Exemple D | 19,9 | <0,1 | 537 | 1,11 |

(suite)

| Exemples | Caractéristiques mesurées des capsules | | | |
|---|---|---|---|---|
| | Taux de parfum encapsulé (%) | Taux de parfum libre (%) | Densité de poudre versée (g/l) | Densité absolue |
| Exemple E | 38 | 0,8 | 482 | 1,08 |
| Exemple F | 21,0 | 0,2 | 595 | 1,11 |
| Exemple G | 20,7 | 0,2 | 521 | 1,15 |
| Exemple H (hors invention) | 19,2 | 0,1 | 568 | 1,12 |

## Exemple I comparatif

On a réalisé des capsules ayant la même composition que l'exemple A tel décrit ci-dessus selon le procédé de l'exemple 1 du brevet US6200949 comprenant un séchage par spray-drying (atomisation) de l'émulsion décrite à l'exemple A

L'émulsion est séchée par spray-drying au moyen d'un appareil du type Bowen Lab Model Dryer utilisant de l'air avec un débit de 420m$^3$/h à une température de 204°C et une température externe de 93°C et une vitesse de la turbine de 50000tr/mn.

Aspect morphologique des particules obtenues : polymorphe avec agrégats

## Exemple J comparatif

On a réalisé des capsules ayant la même composition que l'exemple A tel décrit ci-dessus selon le procédé de l'exemple 1 du du brevet US5508259 comprenant un séchage par spray-drying (atomisation) de l'émulsion décrite à l'exemple A.

Le mélange a été séché par spray-drying avec un appareil du type CCM Sulzer avec un débit d'émulsionde 50kg/h, de l'air à un débit de 320m$^3$/h à 350°C et 0,45 bar.

Aspect morphologique des particules obtenues : polymorphe avec agrégats

| Composition | Caractéristiques mesurées des capsules | | | |
|---|---|---|---|---|
| | Taux de parfum encapsulé (%) | Taux de parfum libre (%) | Densité de poudre versée (g/l) | Densité absolue |
| Exemple I (hors invention) | 18,3 | 2,7 | 259 | 1,16 |
| Exemple J (hors invention) | 11,2 | 1,7 | 269 | 1,12 |

## Exemple 1: Produit déodorant et anti-transpirant

On a préparé un aérosol anti-transpirant anhydre ayant la composition suivante :

| Ingrédients | (% en poids) |
|---|---|
| Dimethicone | 23,51 |
| Palmitate d'isopropyle | 4,85 |
| Dimethicone dimethiconol | 8,90 |
| Citrate de triéthyle | 5,66 |
| Bentonite modifiée | 2,10 |
| Hydroxychlorure d'aluminium anhydre | 28,30 |

(suite)

| Ingrédients | (% en poids) |
|---|---|
| Capsules de parfum de l'exemple A | 26,68 |
| Total | 100,00 |

On ajoute dans une cuve équipée d'un agitateur et d'une turbine de fond 35,3g de dimethicone, 7,3g de palmitate d'isopropyle, 13,3g de dimethicone/dimethiconol et 8,5g de citrate de triéthyle. On homogénéise le mélange sous agitation vive (1000tr/mn). Puis on ajoute 3,1g de bentonite modifiée et on laisse au repos pendant 10 minutes. On homogénéise à nouveau le mélange sous agitation vive (1200tr/mn) avant d'ajouter 42,4g d'hydroxychlorure d'aluminium anhydre. On homogénéise encore le mélange sous agitation vive (1200tr/mn) avant d'ajouter 40,0g de capsules de l'exemple A. On a ainsi obtenu un liquide blanc-beige épais opaque qui est introduit dans un flacon d'aérosol fermé. On élimine l'air du récipient puis le remplit avec le gaz propulseur (isobutane) dans le rapport 15 (jus)/85(gaz).

## Exemples C1 et C2 : Produits déodorants et anti-transpirants

Similairement à l'exemple 1, on a préparé des aérosols anti-transpirants anhydres ayant les compositions suivantes:

| Exemple C1 | |
|---|---|
| Ingrédients | (% en poids) |
| Dimethicone | 23,51 |
| Palmitate d'isopropyle | 4,85 |
| Dimethicone dimethiconol | 8,90 |
| Citrate de triéthyle | 5,66 |
| Bentonite modifiée | 2,10 |
| Hydroxychlorure d'aluminium anhydre | 28,30 |
| Capsules de parfum de l'exemple I | 26,68 |
| Total | 100,00 |

| Exemple C2 | |
|---|---|
| Ingrédients | (% en poids) |
| Dimethicone | 23,51 |
| Palmitate d'isopropyle | 4,85 |
| Dimethicone dimethiconol | 8,90 |
| Citrate de triéthyle | 5,66 |
| Bentonite modifiée | 2,10 |
| Hydroxychlorure d'aluminium anhydre | 28,30 |
| Capsules de parfum de l'exemple J | 26,68 |
| Total | 100,00 |

**Evaluation des exemples :**

**Comparaison des formules de l'exemple1 avec les formules des exemples C1 et C2 avant la pressurisation sous gaz**

| Jus d'aérosol | Aspect | Densité* |
|---|---|---|
| Exemple 1 | Liquide épais homogène blanc beige | 1,153 |
| Exemple C1 | Pâte épaisse inhomogène blanc beige | Non mesurable |
| Exemple C2 | Pâte épaisse inhomogène blanc beige | Non mesurable |
| * la densité est mesurée au moyen d'un densimètre Mettler Toledo DA-100M. A l'aide d'une seringue de 5ml, on a prélevé un échantillon du jus homogénéisé que l'on a introduit dans le densimètre. L'appareil indique automatiquement la densité. | | |

**Comparaison des aérosols : Exemple 1, Exemple C1 et Exemple C2**

| Aérosol | Temps de sédimentation | Aspect du jus | Sprayage (buse de 0.41mm*) |
|---|---|---|---|
| Exemple 1 | 25s | 0 | OK |
| ExempleC1 | 15s | 2 | Bouchages répétitifs de la buse puis fuites de gaz |
| ExempleC2 | 13s | 3 | OK |

Les critères de l'évaluation de temps de sédimentation sont les suivants :

$\geq$ 25s = sédimentation lente

15-24s = sédimentation modérée

$\leq$14s = sédimentation rapide

Les critères pour l'aspect du jus sont les suivants :

0 = pas de particules visibles dans le jus (le jus est complètement translucide)
1 = présence de particules visibles faible dans le jus
2 = présence de particules visibles modérée dans le jus
3 = présence de particules visibles forte dans le jus
4 = présence de particules visibles très forte dans le jus
* référence DSPR119 chez Précision.

**Protocole d'évaluation :**

On agite l'aérosol pendant trois secondes pour l'homogénéiser. Puis on pulvérise pendant 2 secondes sur une mouillette (référence chez Granger Veyron : 40140BCSI de taille 4cm x 14cm) de façon à déposer environ 0,07g de composition. Après une minute on évalue l'intensité olfactive (AV) qu'on note de 0 à 10. Puis on simule la transpiration par un ajout d'eau d'environ 0,1g (trois sprays) sur la composition déposée. On attend 30s puis évalue l'intensité olfactive (AP). 4 heures plus tard, on réévalue à nouveau avant et après pulvérisation de la même quantité d'eau. 20 heures plus tard, on réévalue encore de la même façon, l'intensité avant / après pulvérisation d'eau.

| Aérosol | Intensité odeur T0 | | | Intensité odeur T4h | | | Intensité odeur T24h | | |
|---|---|---|---|---|---|---|---|---|---|
| | AV | AP | Δ | AV | AP | Δ | AV | AP | Δ |
| Exemple 1 | 0 | 6 | 6 | 2,7 | 6,7 | 4,0 | 1,3 | 4,0 | 2,7 |
| Exemple C1 | 6 | 8 | 2 | 6,7 | 8,0 | 1,3 | 4,7 | 5,3 | 0,7 |
| Exemple C2 | 6 | 8 | 2 | 4,7 | 6,3 | 1,7 | 3,0 | 2,3 | 0,7 |
| AV = avant ajout d'eau ; AP = après ajout d'eau ; Δ= intensité olfactive (AV - AP) Echelle d'intensité olf active : 0 à 10 (0= inodore; 10= odeur très intense / saturée). | | | | | | | | | |

On a ainsi observé à T0 que l'aérosol de l'exemple 1 comprenant les capsules de parfum selon l'invention ne présente aucune odeur avant l'ajout d'eau contrairement aux aérosols C1 et C2 (hors invention), ce qui montre que les capsules de parfum dans les aérosols C1 et C2 ne sont pas étanches avant même l'ajout d'eau.

On a également observé, aussi bien à 4h qu'à 24h, que l'aérosol de l'exemple 1 après stimulation à l'eau, conduisait à une odeur plus intense que celle observée pour les aérosols C1 et C2, ce qui montre une libération plus importante de parfum en réponse au stimuli eau

### Exemple 2 : Produit déodorant et anti-transpirant

On a préparé un aérosol anti-transpirant anhydre ayant la composition suivante :

| Ingrédients | (% en poids) |
|---|---|
| Dimethicone | 23,26 |
| Palmitate d'isopropyle | 4,80 |
| Dimethicone dimethiconol | 8,80 |
| Citrate de triéthyle | 5,60 |
| Bentonite modifiée | 2,08 |
| Hydroxychlorure d'aluminium anhydre | 28,00 |
| Capsules de parfum de l'exemple A | 26,40 |
| Parfum libre | 1,06 |
| Total | 100,00 |

Les capsules de parfum de l'exemple A peuvent être remplacées par les capsules des exemples B à H décrites précédemment.

On ajoute dans une cuve équipée d'un agitateur et d'une turbine de fond, 46,5g de dimethicone, 9,6 g de palmitate d'isopropyle, 17,6g de dimethicone/dimethiconol, 11,2g de citrate de triéthyle et 2,13g du parfum. On homogénéise le mélange sous agitation vive (1000tr/mn). Puis on ajoute 4,16g de bentonite modifiée et on laisse reposerpendant 10 minutes. On homogénéise à nouveau le mélange sous agitation vive (1200tr/mn) avant d'ajouter 56,0g d'hydroxychlorure d'aluminium anhydre. On homogénéise encore le mélange sous agitation vive (1200tr/mn) puis ajoute de 52,8g des capsules de l'exemple A. On a ainsi obtenu un liquide beige épais opaque qui est ensuite introduit dans un flacon d'aérosol fermé. On élimine l'air du récipient et on le remplit avec le gaz propulseur (isobutane) dans le rapport 15 (jus)/85(gaz).

On a appliqué environ 1,0 g de compoisition de l'exemple 2 sur la peau. Après une minute on a évalué l'intensité de l'odeur de parfum (APPLICATION) en la notant de 0 à 10. Puis 2, 4 et 6 heures plus tard on a réévalué à nouveau l'intensité de l'odeur de parfum (AV) avant un ajout d'eau d'environ 0,1 g (trois sprays) sur la composition appliquée sur la peau. On a attendu 30 secondes puis réévalué l'intensité de l'odeur de parfum (AP).

| Produit | Intensité odeur T0 APPLICATION | Intensité odeur T2h | | | Intensité odeur T4h | | | Intensité odeur T6h | | |
|---|---|---|---|---|---|---|---|---|---|---|
| | | AV | AP | Δ | AV | AP | Δ | AV | AP | Δ |
| **Exemple 2** | **7,0** | **3,75** | **7,5** | **3,75** | **3,5** | **6,5** | **3,0** | **2,5** | **4,5** | **2,0** |

AV = avant ajout d'eau ; AP = après ajout d'eau ;
Δ= amplitude de différence d'intensité odeur de parfum (AV - AP)
Echelle d'intensité odeur de parfum : 0 à 10 (0= inodore; 10= odeur très intense / saturée).

On a ainsi observé à T0 que la composition de l'exemple 2 présente une odeur forte qui diminue rapidement à T 2h T4h et T6h après l'application. On a également observé que la pulvérisation d'eau sur le produit à T2h, T4h et T6h conduit à une augmentation de l'intensité de l'odeur (notamment des notes fraiches), ce qui montre une libération importante de parfum à chaque temps.

**Exemple 3: Produit shampoing sec**

On a préparé un shampoing sec anhydre ayant la composition suivante :

| Ingrédients | (% en poids) |
|---|---|
| Carbonate de calcium | 14.8 |
| Amidon de maïs DRY-FLO (Akzo Nobel) | 75.0 |
| Hectorite modifiée distéaryl ammonium | 2.0 |
| Myristate d'isopropyle | 3.2 |
| Capsules de parfum de l'exemple F | 5.0 |
| Total | 100,00 |

Les capsules de parfum de l'exemple F peuvent être remplacées par les capsules des exemples A à E et G, H décrites précédemment.

On introduit 14,8g de carbonate de calcium, 75,0g d'amidon de maïs, 2,0g d'hectorite modifiée et 3,2g de myristate d'isopropyle dans une cuve équipée d'un agitateur et d'une turbine de fond. On turbine et agite pendant 3min à 1500tr/mn. Puis on ajoute 5,0g de capsules de l'exemple F en homogénéisant le mélange. Le mélange ainsi obtenu est introduit dans un dispositif d'aérosol. On élimine l'air du récipient puis on le remplit avec le gaz propulseur (isobutane) dans le rapport 15 (jus)/85(gaz).

Après pulvérisation de la composition sur les cheveux, on constate lorsque le sujet transpire ou au contact du sébum qu'une libération du parfum se produit au cours de la journée.

**Exemple 4 : Produit shampoing sec**

On a préparé un shampoing sec anhydre ayant la composition suivante :

| Ingrédients | (% en poids) |
|---|---|
| Carbonate de calcium | 14.8 |
| Amidon de maïs DRY-FLO (Akzo Nobel) | 76.4 |
| Hectorite modifiée distéaryl ammonium | 2.0 |
| Myristate d'isopropyle | 3.0 |
| Capsules de parfum de l'exemple B | 3.0 |
| Parfum libre | 0.8 |
| Total | 100,00 |

Les capsules de parfum de l'exemple B peuvent être remplacées par les capsules des exemples A et C à H décrites précédemment.

On ajoute 14,8g de carbonate de calcium, 76,4g d'amidon de maïs, 2,0g d'hectorite modifiée, 3.0g du myristate d'isopropylé et 0,8g du parfum libre dans une cuve équipée d'un agitateur et d'une turbine de fond. On turbine et agite pendant 3 min à 1500 tr/min avec les pales et turbine. Puis on ajoute 3,0g de capsules de l'exemple B en homogénéisant le mélange avec des pâles. Le mélange obtenu est introduit dans un dispositif aérosol. On élimine l'air du récipient et on le remplit avec le gaz propulseur (isobutane) en rapport 15 (jus)/85(gaz).

Après pulvérisation de la composition sur les cheveux, on constate lorsque le sujet transpire ou au contact du sébum qu'une libération du parfum se produit au cours de la journée.

### Exemple 5: Produit déodorant et anti-transpirant

On a préparé un aérosol anti-transpirant anhydre ayant la composition suivante :

| Ingrédients | (% en poids) |
|---|---|
| Dimethicone | 23,26 |
| Palmitate d'isopropyle | 4,80 |
| Dimethicone dimethiconol | 8,80 |
| Citrate de triéthyle | 5,60 |
| Bentonite modifiée | 2,08 |
| Hydroxychlorure d'aluminium anhydre | 28,00 |
| Capsules de parfum de l'exemple H | 26,40 |
| Parfum libre | 1,06 |
| Total | 100,00 |

Les capsules de parfum de l'exemple H peuvent être remplacées par les capsules des exemples A à G décrites précédemment.

On ajoute dans une cuve équipée d'un agitateur et d'une turbine de fond, 46,5g de dimethicone, 9,6 g de palmitate d'isopropyle, 17,6g de dimethicone/dimethiconol, 11,2g de citrate de triéthyle et 2,13g du parfum. On homogénéise le mélange sous agitation vive (1000tr/mn). Puis on ajoute 4,16g de bentonite modifiée et on laisse reposer pendant 10 minutes. On homogénéise à nouveau le mélange sous agitation vive (1200 tr/mn) avant d'ajouter 56,0g d'hydroxychlorure d'aluminium anhydre. On homogénéise encore le mélange sous agitation vive (1200 tr/mn) puis ajoute de 52,8g des capsules de l'exemple H. On a ainsi obtenu un liquide beige épais opaque qui est ensuite introduit dans un flacon d'aérosol fermé. On élimine l'air du récipient et on le remplit avec le gaz propulseur (isobutane) dans le rapport 15 (jus)/85(gaz).

Après pulvérisation de la composition sur les aisselles, on constate qu'au contact de la sueur, le parfum de la composition déposée se libère et l'effet olfactif perdure toute la journée.

### Revendications

**1.** Composition anhydre comprenant :

1) au moins des particules comprenant un cœur contenant au moins un agent bénéfique et une enveloppe entourant le cœur ; ladite enveloppe comprenant au moins un polysaccharide modifié hydrophobe choisi parmi les $C_5$-$C_{20}$-alcényl succinates d'amidon et au moins un glucide hydrosoluble choisi parmi les maltodextrines ayant un Dextrose Equivalent allant de 4 à 20 ;

lesdites particules présentant simultanément une densité de poudre versée allant de 300,0 g/l à 600,0 g/l et une densité absolue supérieure à 1.0, et

les particules étant sphériques et, ayant un diamètre moyen en nombre allant de 1 à 30$\mu$m et un diamètre moyen en volume allant de 5 à 150$\mu$m, et

2) au moins un agent propulseur.

**2.** Composition selon la revendication 1, comprenant un milieu physiologiquement acceptable.

**3.** Composition selon la revendication 1 ou 2, où les particules sont sphériques et, en particulier ont un diamètre moyen en nombre allant de 2 à 15 μm et de préférence de 5 à 10 μm et un diamètre moyen en volume allant de 10 à 100 μm et de préférence de 20 à 80 μm.

**4.** Composition selon l'une quelconque des revendications 1 à 3, où le polysaccharide modifié hydrophobe est l'octényle succinate d'amidon sodique.

**5.** Composition selon l'une quelconque des revendications 1 à 4, où le polysaccharide modifié hydrophobe représente de 25 à 80% en poids, notamment 30 à 65% en poids, et mieux 40 à 60% en poids, par rapport au poids total de l'enveloppe de la particule.

**6.** Composition selon l'une quelconque des revendications précédentes, où le glucide hydrosoluble est choisi parmi les maltodextrines de DE allant de 12 à 20.

**7.** Composition selon l'une quelconque des revendications précédentes, où le ou les glucides (s) hydrosoluble(s) représentent de préférence de 20 à 75% en poids, plus préférentiellement de 25 à 65% en poids, et mieux de 40 à 60% en poids par rapport au poids total de l'enveloppe de la particule

**8.** Composition selon l'une quelconque des revendications précédentes, où l'enveloppe des particules à libération d'agent bénéfique est constituée

a) d'au moins un $C_5$-$C_{20}$-alcenyl succinate d'amidon dans une quantité allant de 25 à 80% en poids, notamment 30 à 65% en poids, et mieux 40 à 60% en poids, par rapport au poids total de l'enveloppe de la particule et
b) moins une maltodextrine de D.E. allant de 4 à 20 et de préférence allant de 12 à 20, dans une quantité allant de 20 à 75% en poids, plus préférentiellement de 25 à 65% en poids, et mieux de 40 à 60% en poids par rapport au poids total de l'enveloppe de la particule.

**9.** Composition selon l'une quelconque des revendications précédentes, où les particules à libération d'agent bénéfique sont susceptibles d'être obtenues selon un procédé comprenant au moins les étapes suivantes :

- on prépare une solution aqueuse constituée du mélange du glucide hydrosoluble et du polysaccharide modifié hydrophobe puis on ajoute l'agent bénéfique et on agite de façon à former une émulsion ; et
- on homogénéise ladite émulsion ainsi formée sous haute pression à une pression allant de 10 à 200 bars et plus préférentiellement de 20 à 200 bars ; et
- on pulvérisée ladite émulsion dans une chambre de séchage, et
- l'eau est extraite pendant une durée de préférence ne dépassant pas 3 heures, et plus préférentiellement ne dépassant pas 30 minutes, avec un fluide sous pression tel que le dioxyde de carbone, de préférence à l'état supercritique de préférence à une pression d'au moins 0,3XPc et à une température d'au moins Tc-60°C avec Pc correspondant à la pression critique du gaz et Tc la température critique du gaz, de façon à obtenir les particules à libération d'agent bénéfique.

**10.** Composition selon l'une quelconque des revendications précédentes, où les agents bénéfiques sont choisis parmi :

(i) les corps gras ;
(ii) les substances parfumantes ;
(iii) les principes actifs pharmaceutiques ;
(iv) les actifs cosmétiques.

**11.** Composition selon l'une quelconque des revendications précédentes, où les agents bénéfiques sont choisis parmi les substances parfumantes et plus particulièrement celles choises parmi les notes de cœur et/ou de tête et encore plus particulièrement choisies parmi :

Acétate de benzyle
Acétate de géranyle
Acétate de cis-3-hexenyle
Aldéhyde C18 ou nonalactone

Acétate de décyle

Allyl amyl glycolate (citral)

Acétate d'éthyle

Acétate de butyle

Allyl 3-cyclohexylpropionate

Acétate de linalyle

Alcool Phényléthylique

Acétate d'héxyle

Berryflor ou éthyl-6-(acétyloxy)-hexanoate

Acétate d'isoamyle

Caproate d'allyle

Amarocite ou 6,6-diméthoxy-2,5,5-triméthylhex-2-ène Citral lemarome N ou 3,7-diméthylocta-2,6-diénal

Canthoxal ou anisyl propanal

Claritone ou 2,4,4,7-tétraméthyloct-6-en-3-one

Ethyl-2méthyl butyrate

Dihydromyrcénol

Cis-3 hexenol

Hédione ou méthyl dihydrojasmonate

L-carvone

Heptanoate d'allyle

Limonène

Néobuténone alpha ou 1-(5,5-diméthyl-1-cyclohexenyl)-pent-4-èn-1-one

Méthylheptènone

Toscanol ou 4-(cyclopropylméthyl)-phénylméthyl éther myrcenol super ou 2-methyl-6-methylideneoct-7-en-2-ol Decalactone

Acétate de stéaryle

Oxyde de rose

Linalool

Triplal ou 2,4-dimethylcyclohex-3-ène-1-carbaldéhyde Mélonal ou 2,6-dimethylhept-5-ènal

1-(1,2,3,4,5,6,7,8-octahydro-2,3,8,8-tetraméethyl-2-naphthyl)éthan-1-one

Hexylcinnamal

Tétrahydro-2-isobutyl-4-méthylpyrann-4-ol

Salicylate d'hexyle

1,4-Dioxacycloheptadécane-5,17-dione

et leurs mélanges

12. Composition selon l'une quelconque des revendications précédentes, où les particules comprennent au moins une ou plusieurs substances parfumantes ayant une pression de vapeur saturante à 25°C supérieure ou égale à 10,0 Pa et préférentiellement la ou lesdites substances parfumantes représentent de 50 à 100% en poids, encore plus préférentiellement de 60 à 100% en poids, plus particulièrement de 70 à 100% en poids, et encore mieux de 80 à 100% en poids du poids total des substances parfumantes présentes dans les particules

13. Composition selon l'une quelconque des revendications précédentes, **caractérisée par le fait que**

a) les particules comprennent au moins une substance parfumante et
b) la composition comprend en plus au moins une substance parfumante sous forme libre, identique ou différente de ladite substance parfumante présente dans lesdites particules.

14. Composition selon l'une quelconque des revendications précédentes, **caractérisée par le fait qu'**elle contient exclusivement une ou plusieurs substances parfumantes encapsulées dans les particules.

15. Composition selon l'une quelconque des revendications précédentes comprenant au moins un actif déodorant et/ou au moins un actif anti-transpirant sous forme libre et/ou sous forme encapsulée et dans laquelle, plus particulièrement, les particules comprennent au moins une substance parfumante, et encore plus particulièrement, où la composition comprend en plus au moins une substance parfumante sous forme libre, identique ou différente de la substance parfumante présente dans lesdites particules.

**16.** Dispositif aérosol constitué par un récipient comprenant une composition telle que définie selon l'une quelconque des revendications précédentes et par un moyen de distribution de ladite composition.

**17.** Procédé cosmétique de soin et/ou d'hygiène et/ou de de conditionnement et/ou de parfumage et/ou de maquillage d'une matière kératinique consistant à appliquer sur ladite matière kératinique une composition selon l'une quelconque des revendications 1 à 15.

**18.** Procédé cosmétique de traitement des odeurs corporelles et éventuellement de la transpiration humaine consistant à appliquer sur une matière kératinique une composition selon la revendication 15.

**19.** Produit de consommation, **caractérisé par le fait qu'**il est conditionné dans un dispositif aérosol constitué par un récipient comprenant une composition telle que définie selon l'une quelconque des revendications 1 à 15 et par un moyen de distribution de ladite composition.

**Patentansprüche**

**1.** Wasserfreie Zusammensetzung, umfassend

1) mindestens Teilchen mit einem Kern, der mindestens einen Effektstoff enthält, und einer den Kern umgebenden Hülle; wobei die Hülle mindestens ein hydrophob modifiziertes Polysaccharid, das aus Stärke-$C_5$-$C_{20}$-alkenylsuccinaten ausgewählt ist, und mindestens ein wasserlösliches Kohlenhydrat, das aus Maltodextrinen mit einem Dextroseäquivalent im Bereich von 4 bis 20 ausgewählt ist, umfasst; wobei die Teilchen gleichzeitig eine Pulverschüttdichte im Bereich von 300,0 g/l bis 600,0 g/l und eine absolute Dichte von mehr als 1,0 aufweisen; wobei die Teilchen kugelförmig sind und einen zahlenmittleren Durchmesser im Bereich von 1 bis 30 μm und einen volumenmittleren Durchmesser im Bereich von 5 bis 150 μm aufweisen; und 2) mindestens ein Treibmittel.

**2.** Zusammensetzung nach Anspruch 1, umfassend ein physiologisch unbedenkliches Medium.

**3.** Zusammensetzung nach Anspruch 1 oder 2, wobei die Teilchen kugelförmig sind und insbesondere einen zahlenmittleren Durchmesser im Bereich von 2 bis 15 μm und vorzugsweise von 5 bis 10 μm und einen volumenmittleren Durchmesser im Bereich von 10 bis 100 μm und vorzugsweise von 20 bis 80 μm aufweisen.

**4.** Zusammensetzung nach einem der Ansprüche 1 bis 3, wobei es sich bei dem hydrophob modifizierten Polysaccharid um Natriumstärkeoctenylsuccinat handelt.

**5.** Zusammensetzung nach einem der Ansprüche 1 bis 4, wobei das hydrophob modifizierte Polysaccharid 25 bis 80 Gew.-%, insbesondere 30 bis 65 Gew.-% und besser 40 bis 60 Gew.-%, bezogen auf das Gesamtgewicht der Hülle des Teilchens, ausmacht.

**6.** Zusammensetzung nach einem der vorhergehenden Ansprüche, wobei das wasserlösliche Kohlenhydrat aus Maltodextrinen mit einem Dextroseäquivalent im Bereich von 12 bis 20 ausgewählt ist.

**7.** Zusammensetzung nach einem der vorhergehenden Ansprüche, wobei das wasserlösliche Kohlenhydrat bzw. die wasserlöslichen Kohlenhydrate vorzugsweise 20 bis 75 Gew.-%, weiter bevorzugt 25 bis 65 Gew.-% und besser 40 bis 60 Gew.-%, bezogen auf das Gesamtgewicht der Hülle des Teilchens, ausmacht bzw. ausmachen.

**8.** Zusammensetzung nach einem der vorhergehenden Ansprüche, wobei die Hülle der Teilchen mit Freisetzung von Effektstoff aus

a) mindestens einem Stärke-$C_5$-$C_{20}$-alkenylsuccinat in einer Menge im Bereich von 25 bis 80 Gew.-%, insbesondere 30 bis 65 Gew.-% und besser 40 bis 60 Gew.-%, bezogen auf das Gesamtgewicht der Hülle des Teilchens, und b) mindestens einem Maltodextrin mit einem D.E. im Bereich von 4 bis 20 und vorzugsweise im Bereich von 12 bis 20 in einer Menge im Bereich von 20 bis 75 Gew.-%, weiter bevorzugt von 25 bis 65 Gew.-% und besser von 40 bis 60 Gew.-%, bezogen auf das Gesamtgewicht der Hülle des Teilchens,

besteht.

9. Zusammensetzung nach einem der vorhergehenden Ansprüche, wobei die Teilchen mit Freisetzung von Effektstoff gemäß einem Verfahren erhältlich sind, das mindestens die folgenden Schritte umfasst:

- man stellt eine wässrige Lösung her, die aus der Mischung des wasserlöslichen Kohlenhydrats und des hydrophob modifizierten Polysaccharids besteht, gibt dann den Effektstoff zu und rührt zur Bildung einer Emulsion; und
- man homogenisiert die so gebildete Emulsion unter hohem Druck bei einem Druck im Bereich von 10 bis 200 bar und weiter bevorzugt von 20 bis 200 bar; und
- man versprüht die Emulsion in einer Trocknungskammer; und
- man extrahiert das Wasser über einen Zeitraum, der vorzugsweise nicht mehr als 3 Stunden und weiter bevorzugt nicht mehr als 30 Minuten beträgt, mit einem unter Druck stehenden Fluid wie Kohlendioxid, vorzugsweise in überkritischem Zustand, vorzugsweise bei einem Druck von mindestens 0,3xPc und einer Temperatur von mindestens Tc-60 °C, wobei Pc dem kritischen Druck des Gases entspricht und Tc dem kritischen Druck des Gases entspricht, wodurch man die Teilchen mit Freisetzung von Effektstoff erhält.

10. Zusammensetzung nach einem der vorhergehenden Ansprüche, wobei die Effektstoffe aus

(i) Fettsubstanzen;
(ii) Parfümierungssubstanzen;
(iii) pharmazeutischen Wirkstoffen;
(iv) kosmetischen Wirkstoffen

ausgewählt sind.

11. Zusammensetzung nach einem der vorhergehenden Ansprüche, wobei die Effektstoffe aus Parfümierungssubstanzen ausgewählt sind und spezieller aus jenen, die aus Herz- und/oder Kopfnoten und noch spezieller aus
Benzylactetat
Geranylacetat
cis-3-Hexenylacetat
$C_{18}$-Aldehyd oder Nonalacton
Decylacetat
Allylamylglykolat (Citral)
Ethylacetat
Butylactetat
Allyl-3-cyclohexylpropionat
Linalylacetat
Phenylethylalkohol
Hexylacetat
Berryflor oder Ethyl-6-(acetyloxy)hexanoat Isoamylacetat
Allylcaproat
Amarocite oder 6,6-Dimethoxy-2,5,5-trimethylhex-2-en
Citral lemarome N oder 3,7-Dimethylocta-2,6-dienal Canthoxal oder Anisylpropanal
Claritone oder 2,4,4,7-Tetramethyloct-6-en-3-on Ethyl-2-methylbutyrat
Dihydromyrcenol
cis-3-Hexenol
Hedion oder Methyldihydrojasmonat L-Carvon
Allylheptanoat
Limonen
Neobutenon alpha oder 1-(5,5-Dimethyl-1-cyclohexenyl)pent-4-en-1-on
Methylheptenon
Toscanol oder 4-(Cyclopropylmethyl)phenylmethyl-ether
Myrcenol super oder 2-Methyl-6-methylidenoct-7-en-2-ol
Decalacton
Stearylacetat
Rosenoxid
Linalool

Triplal oder 2,4-Dimethylcyclohex-3-en-1-carbaldehyd
Melonal oder 2,6-Dimethylhept-5-enal 1-(1,2,3,4,5,6,7,8-Octahydro-2,3,8,8-tetramethyl-2-naphthyl)ethan-1-on
Hexylcinnamal
Tetrahydro-2-isobutyl-4-methylpyran-4-ol Hexylsalicylat
1,4-Dioxacycloheptadecan-5,17-dion
und Mischungen davon
ausgewählt sind.

**12.** Zusammensetzung nach einem der vorhergehenden Ansprüche, wobei die Teilchen mindestens eine oder mehrere Parfümierungssubstanzen mit einem Sättigungsdampfdruck bei 25 °C größer oder gleich 10,0 Pa aufweisen und vorzugsweise die Parfümierungssubstanz bzw. die Parfümierungssubstanzen 50 bis 100 Gew.-%, weiter bevorzugt 60 bis 100 Gew.-%, noch weiter bevorzugt 70 bis 100 Gew.-% und noch besser 80 bis 100 Gew.-% des Gesamtgewichts der in den Teilchen vorliegenden Parfümierungssubstanzen ausmacht bzw. ausmachen.

**13.** Zusammensetzung nach einem der vorhergehenden Ansprüche, **dadurch gekennzeichnet, dass**

a) die Teilchen mindestens eine Parfümierungssubstanz umfassen und
b) die Zusammensetzung außerdem mindestens eine Parfümierungssubstanz in freier Form, die mit der in den Teilchen vorliegenden Parfümierungssubstanz identisch oder davon verschieden ist, umfasst.

**14.** Zusammensetzung nach einem der vorhergehenden Ansprüche, **dadurch gekennzeichnet, dass** sie ausschließlich eine oder mehrere in den Teilchen verkapselte Parfümierungssubstanzen enthält.

**15.** Zusammensetzung nach einem der vorhergehenden Ansprüche, umfassend mindestens einen Deodorantwirkstoff und/oder mindestens einen Antitranspirantwirkstoff in freier Form und/oder in verkapselter Form, und spezieller wobei die Teilchen mindestens eine Parfümierungssubstanz umfassen und noch spezieller wobei die Zusammensetzung außerdem mindestens eine Parfümierungssubstanz in freier Form, die mit der in den Teilchen vorliegenden Parfümierungssubstanz identisch oder davon verschieden ist, umfasst.

**16.** Aerosolvorrichtung, die aus einem Behälter, der eine Zusammensetzung gemäß einem der vorhergehenden Ansprüche umfasst, und einem Mittel zum Verteilen der Zusammensetzung besteht.

**17.** Kosmetisches Verfahren zur Pflege und/oder zur Hygiene und/oder zur Konditionierung und/oder zur Parfümierung und/oder zum Schminken eines Keratinmaterials, das darin besteht, dass man auf das Keratinmaterial eine Zusammensetzung nach einem der Ansprüche 1 bis 15 aufbringt.

**18.** Kosmetisches Verfahren zur Behandlung von Körpergerüchen und gegebenenfalls menschlicher Transpiration, das darin besteht, dass man eine Zusammensetzung nach Anspruch 15 auf ein Keratinmaterial aufbringt.

**19.** Konsumprodukt, **dadurch gekennzeichnet, dass** es in einer Aerosolvorrichtung, die aus einem Behälter, der eine Zusammensetzung gemäß einem der Ansprüche 1 bis 15 umfasst, und einem Mittel zum Verteilen der Zusammensetzung besteht, verpackt ist.

**Claims**

**1.** Anhydrous composition comprising:

1) at least particles comprising a core containing at least one beneficial agent and a shell surrounding the core; the said shell comprising at least one hydrophobic-modified polysaccharide chosen from starch $C_5$-$C_{20}$-alkenyl succinates and at least one water-soluble carbohydrate chosen from maltodextrins having a Dextrose Equivalent ranging from 4 to 20; the said particles simultaneously exhibiting a poured powder density ranging from 300.0 g/l to 600.0 g/l and an absolute density of greater than 1.0, and the particles being spherical and having a number-average diameter ranging from 1 to 30 $\mu$m and a volume-average diameter ranging from 5 to 150 $\mu$m, and
2) at least one propellant.

2. Composition according to Claim 1, comprising a physiologically acceptable medium.

3. Composition according to Claim 1 or 2, where the particles are spherical and in particular have a number-average diameter ranging from 2 to 15 $\mu$m and preferably from 5 to 10 $\mu$m and a volume-average diameter ranging from 10 to 100 $\mu$m and preferably from 20 to 80 $\mu$m.

4. Composition according to any one of Claims 1 to 3, where the hydrophobic-modified polysaccharide is starch sodium octenyl succinate.

5. Composition according to any one of Claims 1 to 4, where the hydrophobic-modified polysaccharide represents from 25% to 80% by weight, in particular from 30% to 65% by weight and better still from 40% to 60% by weight, with respect to the total weight of the shell of the particle.

6. Composition according to any one of the preceding claims, where the water-soluble carbohydrate is chosen from maltodextrins with a DE ranging from 12 to 20.

7. Composition according to any one of the preceding claims, where the water-soluble carbohydrate(s) preferably represent from 20% to 75% by weight, more preferentially from 25% to 65% by weight and better still from 40% to 60% by weight, with respect to the total weight of the shell of the particle.

8. Composition according to any one of the preceding claims, where the shell of the particles having release of beneficial agent is formed:

   a) of at least one starch $C_5$-$C_{20}$-alkenyl succinate in an amount ranging from 25% to 80% by weight, in particular from 30% to 65% by weight and better still from 40% to 60% by weight, with respect to the total weight of the shell of the particle, and
   b) at least one maltodextrin with a DE ranging from 4 to 20 and preferably ranging from 12 to 20, in an amount ranging from 20% to 75% by weight, more preferentially from 25% to 65% by weight and better still from 40% to 60% by weight, with respect to the total weight of the shell of the particle.

9. Composition according to any one of the preceding claims, where the particles having release of beneficial agent are capable of being obtained according to a process comprising at least the following stages:

   - an aqueous solution constituted of the mixture of the water-soluble carbohydrate and of the hydrophobic-modified polysaccharide is prepared, then the beneficial agent is added and stirring is carried out so as to form an emulsion; and
   - the said emulsion thus constituted is homogenized under high pressure at a pressure ranging from 10 to 200 bar and more preferentially from 20 to 200 bar; and
   - the said emulsion is sprayed into a drying chamber; and
   - the water is extracted for a period of time preferably not exceeding 3 hours and more preferentially not exceeding 30 minutes, with a pressurized fluid, such as carbon dioxide, preferably in the supercritical state, preferably at a pressure of at least 0.3 X Pc and at a temperature of at least Tc-60ºC, with Pc corresponding to the critical pressure of the gas and Tc to the critical temperature of the gas, so as to obtain the particles having release of beneficial agent.

10. Composition according to any one of the preceding claims, where the beneficial agents are chosen from:

   (i) fatty substances;
   (ii) fragrancing substances;
   (iii) pharmaceutical active principles;
   (iv) cosmetic active principles.

11. Composition according to any one of the preceding claims, where the beneficial agents are chosen from fragrancing substances and more particularly those chosen from the heart and/or top notes and more particularly still chosen from:

   Benzyl acetate
   Geranyl acetate
   cis-3-Hexenyl acetate

C$_{18}$ aldehyde or nonalactone
Decyl acetate
Allyl amyl glycolate (citral)
Ethyl acetate
Butyl acetate
Allyl 3-cyclohexylpropionate
Linalyl acetate
Phenylethyl alcohol
Hexyl acetate
Berryflor or ethyl 6-(acetyloxy)hexanoate
Isoamyl acetate
Allyl caproate
Amarocite or 6,6-dimethoxy-2,5,5-trimethylhex-2-ene
Citral lemarome N or 3,7-dimethylocta-2,6-dienal
Canthoxal or anisyl propanal
Claritone or 2,4,4,7-tetramethyloct-6-en-3-one
Ethyl 2-methylbutyrate
Dihydromyrcenol
cis-3-Hexanol
Hedione or methyl dihydrojasmonate
L-Carvone
Allyl heptanoate
Limonene
Neobutenone alpha or 1-(5,5-dimethyl-1-cyclohexenyl)-pent-4-en-1-one
Methylheptanone
Toscanol or 4-(cyclopropylmethyl)phenyl methyl ether
Myrcenol Super or 2-methyl-6-methylideneoct-7-en-2-ol
Decalactone
Stearyl acetate
Rose oxide
Linalool
Triplal or 2,4-dimethylcyclohex-3-ene-1-carbaldehyde
Melonal or 2,6-dimethylhept-5-enal
1-(1,2,3,4,5,6,7,8-octahydro-2,3,8,8-tetramethyl-2-naphthyl)ethan-1-one
Hexylcinnamal
Tetrahydro-2-isobutyl-4-methylpyran-4-ol Hexyl salicylate
1,4-dioxacycloheptadecane-5,17-dione
and their mixtures.

12. Composition according to any one of the preceding claims, where the particles comprise at least one or more fragrancing substances having a saturated vapour pressure at 25ºC of greater than or equal to 10.0 Pa and preferentially the said fragrancing substance(s) represent from 50% to 100% by weight, more preferentially still from 60% to 100% by weight, more particularly from 70% to 100% by weight and better still from 80% to 100% by weight of the total weight of the fragrancing substances present in the particles.

13. Composition according to any one of the preceding claims, **characterized in that**:

a) the particles comprise at least one fragrancing substance and
b) the composition additionally comprises at least one fragrancing substance in the free form, identical to or different from the said fragrancing substance present in the said particles.

14. Composition according to any one of the preceding claims, **characterized in that** it contains exclusively one or more fragrancing substances encapsulated in the particles.

15. Composition according to any one of the preceding claims, comprising at least one deodorant active principle and/or at least one antiperspirant active principle in the free form and/or in the encapsulated form and in which more particularly the particles comprise at least one fragrancing substance and more particularly still where the composition additionally comprises at least one fragrancing substance in the free form, identical to or different from the fragrancing substance present in the said particles.

16. Aerosol device constituted of a container comprising a composition as defined according to any one of the preceding claims and of a means for dispensing the said composition.

17. Cosmetic method for caring for and/or for the hygiene of and/or for conditioning and/or for fragrancing and/or for making up a keratinous substance which consists in applying, to the said keratinous substance, a composition according to any one of Claims 1 to 15.

18. Cosmetic method for the treatment of body odours arid optionally of perspiration of human beings which consists in applying, to a keratinous substance, a composition according to Claim 15.

19. Consumer product, **characterized in that** it is packaged in an aerosol device constituted of a container comprising a composition as defined according to any one of Claims 1 to 15 and of a means for dispensing the said composition.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2008156236 A
- US 2004029750 A1
- US 5508259 A
- US 6200949 B
- EP 1917098 B1
- US 4077441 A
- US 4850517 A
- US 5002698 A
- US 2005063928 A
- US 2005084464 A
- US 2005084474 A
- EP 1658863 A
- US 6916465 B

**Littérature non-brevet citée dans la description**

- *CHEMICAL ABSTRACTS,* 66829-29-6
- **S. ARCTANDER.** *Perfume and Flavor Chemicals,* 1969
- **S. ARCTANDER.** *Perfume and Flavor Materials of Natural Origin,* 1960
- Flavor and Fragrance Materials. Allured Publishing Co, 1991
- *CHEMICAL ABSTRACTS,* 51981-21-6